# EUROPÄISCHE PATENTSCHRIFT

(11) **EP 1 796 557 B1**
(45) Veröffentlichungstag und Bekanntmachung des Hinweises auf die Patenterteilung: **29.06.2016**
(21) Anmeldenummer: 05786845.7
(22) Anmeldetag: 28.09.2005
(51) Int. Cl.: A61B 17/16, A61B 17/32

(54) **CHIRURGISCHES INSTRUMENT**
SURGICAL INSTRUMENT
INSTRUMENT CHIRURGICAL

(30) Priorität: 01.10.2004 DE 102004049247
(43) Veröffentlichungstag der Anmeldung: 20.06.2007
(73) Patentinhaber: Aesculap AG, 78532 Tuttlingen (DE)
(72) Erfinder: SCHULZ, Peter, 79843 Löffingen (DE); WEISSHAUPT, Dieter, 78194 Immendingen (DE); NESPER, Markus, 78532 Tuttlingen (DE); FAULHABER, Konstantin, 78665 Frittlingen (DE); LUTZE, Theodor, 78582 Balgheim (DE)
(74) Vertreter: Hoeger, Stellrecht & Partner Patentanwälte mbB
(86) Internationale Anmeldenummer: PCT/EP2005/010501
(87) Internationale Veröffentlichungsnummer: WO 2006/037542

(56) Entgegenhaltungen:
- US-A- 3 752 161
- US-A- 4 943 294
- US-A- 5 163 598
- US-A- 5 339 723
- US-A- 5 361 583

## Beschreibung

Die vorliegende Erfindung betrifft ein chirurgisches Instrument umfassend einen Handhabungsteil und einen Werkzeugteil, wobei das Werkzeugteil mindestens ein beweglich gelagertes Werkzeug umfaßt, welches über einen Kraftübertragungs- und/oder Betätigungsmechanismus, welcher vom Handhabungsteil aus bedienbar ist, betätigbar ist, und daß der Kraftübertragungs- und/oder Betätigungsmechanismus eine mit einem Fluid betreibbare Antriebseinheit umfaßt, wobei mit der Antriebseinheit in einer ersten Antriebsstellung das beweglich gelagerte Werkzeug mit einer ersten Betätigungskraft beaufschlagbar und in mindestens einer zweiten Antriebsstellung mit mindestens einer zweiten Betätigungskraft beaufschlagbar ist und wobei die Antriebseinheit einen Linearantrieb umfaßt.

Chirurgische Instrumente der eingangs beschriebenen Art sind beispielsweise bekannt in Form von Knochenstanzen zum Entfernen von Knochen, Knorpel oder derartigem Gewebe. Nachteilig bei den bekannten Knochenstanzen ist es, daß durch die mit einem Fluid betreibbare Antriebseinheit das beweglich gelagerte Werkzeug nur bedingt oder sogar überhaupt nicht feinfühlig betätigbar ist. Grund hierfür ist, daß die Antriebseinheit entweder mit einem Fluidstrom bestimmten Druckes beaufschlagt werden kann oder nicht. Dies hat jedoch die nachteilige Konsequenz, daß im Falle einer Betätigung des Betätigungsmechanismus das Instrument in der Regel schlagartig von einer Ruhe- oder Ausgangsstellung in eine Arbeitsstellung überführt wird oder umgekehrt. Dies kann zur Folge haben, daß beispielsweise mit einer Knochenstanze nicht ein gewünschter Gewebebereich, sondern ein danebenliegender entfernt wird.

Aus der US 5,339,723 und der US 5,361,583 sind Betätigungssysteme zur Verstärkung einer Betätigungskraft für chirurgische Instrumente bekannt, die mit einem unter Druck stehenden Fluid betrieben werden. Ein weiteres fluidbetriebenes chirurgisches Instrument ist in der US 3,752,161 offenbart. Dieses Dokument offenbart ein chirurgisches Instrument mit den Merkmalen, die im Oberbegriff des Anspruchs 1 definiert sind. Die US 5,163,598 befasst sich mit einem Sternum-Klammergerät. Und schließlich offenbart die US 4,943,294 ein angetriebenes Gerät zum Markieren von Vieh mittels Ohrplaketten.

Es ist daher Aufgabe der vorliegenden Erfindung, ein chirurgisches Instrument der eingangs beschriebenen Art so zu verbessern, daß es möglichst feinfühlig betrieben werden kann.

Diese Aufgabe wird bei einem chirurgischen Instrument der eingangs beschriebenen Art erfindungsgemäß dadurch gelöst, daß der Linearantrieb zwei gekoppelte, getrennt ansteuerbare Fluidzylinder umfaßt.

Die erfindungsgemäße Weiterbildung bekannter chirurgischer Instrumente hat den Vorteil, daß ein Operateur das Instrument im Einsatz feinfühliger bedienen kann. Insbesondere dann, wenn sich die erste Betätigungskraft von der zweiten Betätigungskraft unterscheidet, kann beispielsweise das Werkzeug in der ersten Antriebsstellung mit einer Kraft beaufschlagt werden, die nicht ausreicht, beispielsweise bei einer Knochenstanze, um Gewebe zu durchtrennen, sondern allenfalls das Werkzeug an das Gewebe heranzuführen. Dies gestattet es dem Operateur, das Instrument in gewünschter Weise zu positionieren und erst dann das Instrument in die zweite Antriebsstellung zu überführen, in welcher das Werkzeug eine Arbeitsstellung einnimmt, in welcher es also beispielsweise Gewebe schneiden kann. Der Aufbau des Instruments wird ferner dadurch vereinfacht, dass die Antriebseinheit einen Linearantrieb umfaßt. Dieser könnte parallel zur Längsrichtung angeordnet sein, vorzugsweise ist er jedoch relativ zur Längsrichtung geneigt angeordnet. Auf diese Weise kann er innerhalb eines Griffs des Handhabungsteils angeordnet werden. So kann das Instrument besonders kompakt und handlich ausgebildet werden, beispielsweise mit einem Handhabungsteil in Form eines Pistolengriffs. Um ganz auf eine elektrische Energieversorgung verzichten zu können, ist es günstig, dass der Linearantrieb mindestens einen Fluidzylinder umfaßt. Fluidzylinder sind besonders wartungsarm und haben in der Regel eine hohe Lebensdauer. Um den Linearantrieb vollständig auf Basis von Fluidzylindern auszubilden, ist es vorteilhaft, dass der Linearantrieb zwei gekoppelte, getrennt ansteuerbare Fluidzylinder umfaßt. Dies erlaubt es, den einen Fluidzylinder unabhängig vom anderen Fluidzylinder mit einem Fluid zum Erzeugen einer Antriebskraft zu beaufschlagen. Genauso können aber auch beide Fluidzylinder gleichzeitig mit einem Fluid beaufschlagt werden, so daß insgesamt drei aktive Antriebsstellungen vorgebbar sind. Wird keiner der beiden Fluidzylinder mit einem Fluid zum Erzeugen einer Antriebskraft beaufschlagt, nimmt das Instrument eine Ruhestellung ein. Diese Stellung wird nachfolgend nicht als Antriebsstellung bezeichnet.

Vorteilhaft ist es, wenn mit der Antriebseinheit in einer dritten Betätigungsstellung das beweglich gelagerte Werkzeug mit einer dritten Betätigungskraft beaufschlagbar ist. Dadurch kann der Operateur das Instrument noch feinfühliger bedienen, da er beispielsweise drei unterschiedliche Betätigungskräfte auf das beweglich gelagerte Werkzeug durch den Kraftübertragungs- und/oder Betätigungsmechanismus ausüben kann.

Vorzugsweise entspricht die dritte Betätigungskraft der Summe der ersten und zweiten Betätigungskraft. Dies gestattet es beispielsweise mit zwei Fluidkolbeneinheiten die Antriebseinheit auszubilden, die jeweils zwei Antriebsstellungen einnehmen können. So wird insbesondere der Aufbau der Antriebseinheit deutlich vereinfacht.

Vorteilhafterweise liegt ein Verhältnis der ersten und zweiten Betätigungskraft im Bereich von 4 : 1 bis 9 : 1. So kann erreicht werden, daß in der ersten Antriebsstellung eine Kraft von etwa 10 bis 20 Prozent der maximal von der Antriebseinheit zur Verfügung stellbaren Kraft auf das bewegliche Werkzeug übertragen wird. Beispielsweise bei einer Knochenstanze reicht dann diese Kraft nicht aus, um zu entfernendes Gewebe abzutrennen.

Um drei verschiedene Betätigungskräfte vorgeben zu können, ist es vorteilhaft, wenn die beiden Fluidzylinder unterschiedliche Wirkungsquerschnitte aufweisen. Sind ihre Wirkungsquerschnitte identisch, so kann entweder keine, die halbe oder die volle von der Antriebseinheit bereitstellbare Antriebskraft erzeugt werden. Unterscheiden sich jedoch die Wirkungsquerschnitte so kann eine erste Antriebskraft kleiner als 50 Prozent der Maximalkraft sein. Die zweite Antriebskraft entspricht dann der Differenz zwischen der ersten und der Maximalkraft und ist somit größer als die Hälfte der Maximalkraft.

Antriebsstellungen mit insbesondere 10 bis 20 Prozent der Maximalkraft, 80 bis 90 Prozent der Maximalkraft und der Maximalkraft lassen sich vorgeben, wenn ein Verhältnis der Wirkungsquerschnitte in einem Bereich von 4 : 1 bis 9 : 1 liegt.

Vorteilhafterweise ist der mindestens eine Fluidzylinder ein Pneumatik- und/oder Hydraulikzylinder. Ein Pneumatikzylinder läßt sich beispielsweise mit Druckluft betreiben, wie sie üblicherweise in jedem Operationssaal zur Verfügung steht. Ferner hat dies den Vorteil, daß auch ein nicht vollständig dichtes System prinzipiell noch betriebsbereit ist. Da zudem nur Luft aus dem System entweichen kann, führt dies zu keiner Beeinträchtigung eines Patienten. Dies könnte allenfalls der Fall sein, wenn Hydraulikzylinder verwendet werden mit einem nicht körperverträglichen Fluid und es zu einer Leckage des Antriebssystems kommt.

Gemäß einer bevorzugten Ausführungsform der Erfindung kann vorgesehen sein, daß der mindestens eine Fluidzylinder ein doppeltwirkender Fluidzylinder ist. Dies gestattet es, einen Kolben des mindestens einen Fluidzylinders von einer Seite mit einem Fluid zu beaufschlagen zum Erzeugen einer gewünschten Antriebskraft. In entgegengesetzter Richtung kann der Kolben ebenfalls mit einem Fluid beaufschlagt werden, um das Instrument wieder in eine Ausgangsstellung zurück zu bewegen und/oder es in der Ausgangsstellung zu halten. Die Ausgangsstellung kann bei einem Instrument beispielsweise derart gewählt werden, daß das beweglich gelagerte Werkzeug eine Offenstellung einnimmt, das heißt von der Ausgangsstellung durch Beaufschlagung mit einer Betätigungskraft in eine Arbeitsstellung überführt wird, in welchem es eine Funktion ausübt, beispielsweise Gewebe schneidet oder hält.

Vorteilhaft ist es, um das Instrument als Knochenstanze zu betreiben, wenn es ausgebildet ist zum Entfernen von Knochen, Knorpel oder derartigem Gewebe, mit einem sich in einer Längsrichtung erstreckenden Schaft, welcher an seinem distalen Ende eine quer zur Längsrichtung angeordnete oder relativ zu dieser geneigte Schneidplatte trägt, wenn das beweglich gelagerte Werkzeug ein am Schaft verschiebbar gelagertes Schneidelement ist, welches an seinem distalen Ende eine in Richtung auf die Schneidplatte weisende Schneide trägt, die an die Schneidplatte heranführbar ist zum Schneiden von Gewebe.

Besonders vorteilhaft ist es, wenn das Werkzeugteil mit dem Handhabungsteil lösbar verbindbar ist. Dies ist besonders einfach möglich, wenn am proximalen Ende des Schafts ein erstes Kupplungselement angeordnet ist zum lösbaren, formschlüssigen Verbinden mit der Kraftübertragungs- und/oder Betätigungseinheit, welche ein zum Kupplungselement korrespondierendes zweites Kupplungselement aufweist, und wenn das erste Kupplungselement ein Vielkant ist, dessen Außenflächen von einer Längsachse des Schafts radial nach außen weisen. Ein Vielkant läßt sich besonders einfach herstellen und gestattet es zudem, das Handhabungsteil am Werkzeugteil in einer Vielzahl diskreter Rotationsstellungen um die Längsachse des Schafts anzuordnen. Dabei entspricht üblicherweise eine Zahl der möglichen Rotationsstellungen der Zahl der Außenflächen des Vielkants.

Vier oder sechs definierte Rotationsstellungen lassen sich vorgeben, wenn der Vielkant ein Vierkant, ein Sechskant oder ein Achtkant ist. Ferner eignet sich die Ausgestaltung als Vielkant dazu, sicherzustellen, daß nur für das Handhabungsteil zugelassene Werkzeugteile mit dem Handhabungsteil verbunden werden können. Das Kupplungselement bildet somit gleichzeitig auch eine Art Kodierelement zum Kodieren eines bestimmten Typs von Werkzeugteil.

Günstig ist es, wenn der Schaft an seinem proximalen Ende hülsenartig geformt ist und wenn das Schneidelement das hülsenartige Ende des Schafts durchsetzt. Der Aufbau des Schafts wird so besonders einfach. Zudem bildet das hülsenartige Ende des Schafts eine Führung in Längsrichtung für das Schneidelement.

Um das Schneidelement auf einfache Weise mit dem Kraftübertragungs- und/oder Betätigungsmechanismus verbinden zu können, ist es vorteilhaft, wenn es an seinem proximalen Ende ein drittes, mit einem Antriebselement des Kraftübertragungs- und /oder Betätigungsmechanismus lösbar verbindbares Kupplungselement trägt und wenn sich an das dritte Kupplungselement in Längsrichtung des Schafts wirkende Anschläge anschließen. Beispielsweise kann das Antriebselement zwischen die in Längsrichtung des Schafts wirkenden Anschläge eingreifen oder, wenn das dritte Kupplungselement in Form eines Vorsprungs ausgebildet ist, die seitlichen Anschläge am Kupplungselement umgreifen oder sogar das Kupplungselement umgreifen. Dadurch kann eine sichere Verbindung und Übertragung der Antriebskraft vom Kraftübertragungs- und/oder Betätigungsmechanismus auf das Schneidelement erreicht werden.

Günstig ist es, wenn in einer Ruhestellung des Instruments, in welcher mit dem Kraftübertragungs- und/oder Betätigungsmechanismus keine Betätigungskraft in einer Betätigungsrichtung auf das Werkzeug ausgeübt wird, mit dem Kraftübertragungs- und/oder Betätigungsmechanismus in einer der Betätigungsrichtung entgegengesetzten Halterichtung eine Haltekraft auf das Werkzeug ausübbar ist. Ohne Betätigung des Kraftübertragungs- und/oder Betätigungsmechanismus ist so sichergestellt, daß das Instrument stets die Ruhestellung einnimmt. Es befindet sich somit immer in einer definierten Ausgangsposition vor einem chirurgischen Einsatz. Auch kann es durch die ausgeübte Haltekraft nicht infolge einer unbeabsichtigten Handhabung, das heißt Halten des Instruments derart, daß das beweglich gelagerte Werkzeug parallel zur Schwerkraftrichtung orientiert ist, aufgrund der wirkenden Schwerkraft bewegt werden.

Vorteilhaft ist es, wenn der Kraftübertragungs- und/oder Betätigungsmechanismus ein Betätigungsglied umfaßt zum Betätigen der Antriebseinheit, wenn das Betätigungsglied von einer unbetätigten Ausgangsstellung, in welcher das Instrument die Ruhestellung einnimmt, in eine Aktivierungsstellung bringbar ist, in welcher die Antriebseinheit die erste Antriebsstellung einnimmt und in mindestens eine zweite Aktivierungsstellung bringbar ist, in welcher die Antriebseinheit die zweite Antriebsstellung einnimmt. Dies gestattet es einem Operateur, das Instrument in gewünschter Weise zu betreiben, nämlich ausgehend von der unbetätigten Ausgangsstellung das Betätigungsglied in eine erste Aktivierungsstellung zu überführen, um das beweglich gelagerte Werkzeug beispielsweise mit einer kleinen Antriebskraft zu beaufschlagen. Er kann aber auch das Betätigungsglied so betätigen, daß das beweglich gelagerte Werkzeug mit einer Maximalkraft beaufschlagt wird. Das Betätigungsglied kann, muß aber nicht zwingend einteilig ausgebildet sein. Denkbar wäre es, das Betätigungsglied insbesondere zu teilen, beispielsweise um zwei Fluidzylinder getrennt anzusteuern, zum Beispiel durch zwei unabhängige Steuerventile, welche ein Operateur, ähnlich wie ein Trompetenspieler, unabhängig voneinander betätigen kann.

Um dem Operateur anzuzeigen, in welcher Position sich das Instrument befindet, ist es günstig, wenn das Betätigungsglied federnd vorgespannt in der Ausgangsstellung gehalten ist. Auf diese Weise weiß ein Operateur immer, wenn er das Instrument in die Hand nimmt, daß es unbetätigt ist.

Ein besonders einfacher Aufbau des Instruments wird erreicht, wenn das Betätigungsglied schwenkbar gelagert ist. Insbesondere kann eine Lagerung um eine Schwenkachse parallel oder quer zur Längsrichtung des Instruments vorgesehen sein.

Gemäß einer weiteren bevorzugten Ausführungsform der Erfindung kann vorgesehen sein, daß in der ersten Aktivierungsstellung eine erste Rückstellkraft auf das Betätigungsglied wirkt, daß in der zweiten Aktivierungsstellung eine zweite Rückstellkraft wirkt und daß die zweite Rückstellkraft größer als die erste Rückstellkraft ist. Auf diese Weise erhält ein Operateur eine taktile Rückmeldung über eine Betätigungsstellung oder Aktivierungsstellung des Betätigungsglieds. Ist beispielsweise die Antriebskraft in der ersten Antriebsstellung kleiner als die Antriebskraft in der zweiten Antriebsstellung, so wird dem Operateur dies durch das Betätigungsglied indirekt mitgeteilt. Ohne auf eine Skala sehen zu müssen, kann ein Operateur erfühlen, welche Antriebsstellung und damit welche Antriebskraft auf das beweglich gelagerte Werkzeug ausgeübt wird.

Um die beiden Aktivierungsstellungen eindeutig unterscheiden zu können, ist es günstig, wenn die zweite Rückstellkraft mindestens doppelt so groß wie die erste Rückstellkraft ist.

Besonders einfach lassen sich Rückstellkräfte erzeugen, wenn zum Erzeugen der ersten und zweiten Rückstellkraft eine Rückstelleinheit umfassend ein erstes elastisches Rückstellglied und ein zweites elastisches Rückstellglied vorgesehen ist. Damit kann jeder Antriebsstellung eine gewünschte Rückstellkraft zugeordnet werden durch Auswahl eines bestimmten elastischen Rückstellglieds.

Günstigerweise ist das erste und/oder das zweite Rückstellglied ein Federelement. Beispielsweise in Form einer Schraubenfeder kann ein zuverlässiges Rückstellglied bereitgestellt werden.

Vorteilhaft kann es ferner sein, wenn die Rückstelleinheit direkt oder indirekt auf ein Steuerglied zum Ansteuern der Antriebseinheit oder auf das Betätigungsglied wirkend ausgebildet ist. Es wird so der Aufbau des Instruments weiter vereinfacht, was einen besonders kompakten Aufbau erlaubt.

Gemäß einer bevorzugten Ausführungsform der Erfindung kann ein Steuerglied zum Ansteuern der Antriebseinheit vorgesehen sein und daß das Betätigungsglied direkt oder indirekt auf das Steuerglied wirkt. Eine solche Ausgestaltung ist insbesondere dann vorteilhaft, wenn ein Betätigungsschaltkreis und ein Arbeitsschaltkreis nicht mit der gleichen Energieform betrieben werden. Beispielsweise kann ein Schaltkreis elektrisch, ein anderer durch ein mit Druck beaufschlagtes Betriebsmittel, beispielsweise ein Fluid, betrieben werden. Dies erfordert eine Umsetzung von Steuersignalen, beispielsweise elektrischen oder mechanischen, in bestimmte Fluidströme oder elektrische Signale für die Antriebseinheit.

Besonders einfach wird der Aufbau des Instruments, wenn das Steuerglied mindestens ein Steuerventil umfaßt. Das Steuerventil kann beispielsweise elektrisch, elektronisch oder mechanisch betätigbar sein. Dies ermöglicht es, ein mechanisches, elektrisches oder elektronisches Betätigungsglied bereitzustellen.

Um drei unterschiedliche Antriebsstellungen realisieren zu können, ist es günstig, wenn das mindestens eine Steuerventil mindestens drei unterschiedliche Schaltstellungen aufweist. Beispielsweise kann so eine Ruhestellung des Instruments sowie eine erste und eine zweite Antriebsstellung realisiert werden, in welcher eine erste und eine zweite Betätigungskraft auf das beweglich gelagerte Werkzeug ausübbar sind.

Vorteilhaft ist es, wenn das Steuerventil einen Ventilstößel aufweist, wenn am Ventilkörper mindestens ein mit einer Fluidquelle verbindbarer Anschluß und für jeden doppeltwirkenden Fluidzylinder zwei Anschlüsse vorgesehen sind, wenn in einer ersten Stellung des Ventilstößels mindestens einer der Fluidzylinder mit Fluid in einer Halterichtung beaufschlagbar ist, wenn in einer zweiten Stellung des Ventilstößels mindestens einer der Fluidzylinder beidseitig mit unterschiedlichen Fluidströmen beaufschlagbar ist und wenn in einer dritten Stellung des Ventilstößels mindestens einer der Fluidzylinder entgegen der Halterichtung mit einem Fluid beaufschlagbar ist. Durch die beidseitige Beaufschlagung mindestens eines der Fluidzylinder in der zweiten Stellung des Ventilstößels kann ein besonders sanfter Übergang von der ersten in die zweite und dann auch in die dritte Stellung des Ventilstößels realisiert werden. Insbesondere ist es möglich, mit einem derartigen Steuerventil einen oder mehrere doppeltwirkende Zylinder mit einer Haltekraft zu beaufschlagen, in einer zweiten Stellung einen der Fluidzylinder mit einem Fluidstrom entgegen der Halterichtung zu beaufschlagen, um eine erste, kleine Betätigungskraft zu erzeugen, und schließlich in der dritten Ventilstellung mehrere oder alle Fluidzylinder mit einem Fluid zu beaufschlagen, um eine maximale Antriebskraft zu erzeugen.

Um möglichst drei unterschiedliche Antriebskräfte mit der Antriebseinheit erzeugen zu können, ist es günstig, wenn in der zweiten Stellung des Ventilstößels ein Fluidstrom auf die beiden Anschlüsse mindestens eines Fluidzylinders ungleich verteilt wird. Dadurch wird der mindestens eine Fluidzylinder definiert mit einer bestimmten Kraftdifferenz in eine vorbestimmte Richtung bewegt und so entweder eine Haltekraft oder eine Antriebskraft erzeugt.

Vorteilhaft ist es, wenn der Ventilstößel mit einer Mehrzahl von Ringnuten versehen ist und in einem Ventilkörper in unterschiedlichen Schaltstellungen unterschiedliche Ringsräume definiert, deren Querschnittsverhältnisse in Abhängigkeit eines Fluiddrucks derart aufeinander angestimmt sind, daß in der zweiten Schaltstellung nur ein Bruchteil einer maximalen Betätigungskraft durch den mindestens eine Fluidzylinder erzeugbar ist. Grundsätzlich wäre es nämlich denkbar, mit einem Steuerventil nur einen einzigen Fluidzylinder zu betreiben, wobei in unterschiedlichen Schaltstellungen des Steuerventils ein doppeltwirkender Fluidzylinder mit unterschiedlichen Druckdifferenzen auf beiden Seiten seines Kolbens beaufschlagt wird, um damit unterschiedliche Antriebskräfte zu erzeugen. Die Ausbildung des Ventilstößels in der beschriebenen Weise, ist leicht herstellbar. Zudem lassen sich so auf einfache Weise gewünschte Antriebskraftverhältnisse vorgeben.

Günstigerweise sind mindestens zwei Steuerventile vorgesehen mit jeweils mindestens zwei Schaltstellungen. Auf diese Weise lassen sich auch insgesamt vier Schaltstellungen, davon eine Ruhestellung, definieren. Zwei Steuerventile haben den Vorteil, daß entweder keines, eines von beiden oder beide gleichzeitig gedrückt werden können. Dadurch weiß ein Operateur auch sofort, in welcher Antriebsstellung sich das Instrument befindet.

Vorteilhafterweise sind die mindestens zwei Steuerventile manuell getrennt betätigbar. So kann ein Operateur direkt ertasten oder erfühlen, in welcher Antriebsstellung sich das Instrument befindet.

Günstig ist es, wenn am Werkzeugteil mindestens eine Kodiereinheit vorgesehen ist zum Kodieren der Art und/oder des Typs des Werkzeugteils und wenn am Handhabungsteil eine Dekodiereinheit vorgesehen ist zum Dekodieren der Art und/oder des Typs des Werkzeugteils. Durch eine Kodierung des Werkzeugteils kann beispielsweise das Handhabungsteil so eingestellt werden, daß eine von der Antriebseinheit erzeugbare Maximalkraft begrenzt wird. Insbesondere dann, wenn empfindliche Werkzeugteile verwendet werden, kann so eine Beschädigung des Werkzeugteils verhindert werden. Ferner wird durch die Kodierung verhindert, daß mit dem Instrument nur zugelassene Werkzeugteile verbunden werden können.

Gemäß einer bevorzugten Ausführungsform der Erfindung kann vorgesehen sein, daß die mindestens eine Kodiereinheit keinen oder mindestens einen am Werkzeugteil abstehenden Vorsprung oder mindestens eine am Werkzeugteil angeordnete Ausnehmung umfaßt, daß die Dekodiereinheit ein zu dem mindestens einen Vorsprung oder der mindestens einen Ausnehmung korrespondierendes Betriebsmodischaltglied umfaßt, daß das Betriebsmodischaltglied mindestens eine erste und eine zweite Betriebsmodistellung aufweist und daß das Betriebsmodischaltglied entsprechend der Kodierung des Werkzeugteils eine der mindestens zwei Betriebsmodistellungen einnimmt. Beispielsweise kann durch das Betriebsmodischaltglied ein Fluidstrom oder eine Energiezufuhr der Antriebseinheit anderweitig begrenzt werden, um eine Beschädigung des Werkzeugteils zu vermeiden.

Es ist daher vorteilhaft, wenn die mindestens zwei Betriebsmodistellungen maximalen Betätigungskräften der Antriebseinheit zugeordnet sind. Beispielsweise kann in einer ersten Betriebsmodistellung eine maximale Antriebskraft der Antriebseinheit erzeugt werden, in einer zweiten Betriebsmodistellung dagegen nur eine reduzierte Antriebskraft, beispielsweise nur eine Antriebskraft, die in etwa 80 bis 90 Prozent der Maximalkraft entspricht.

Günstig ist es, wenn das Betriebsmodischaltglied mit dem Steuerglied gekoppelt ist und wenn das Betriebsmodischaltglied in mindestens einer Betriebsmodistellung mindestens eine Schaltstellung des Steuerglieds direkt oder indirekt außer Kraft setzt. Beispielsweise kann das Betriebsmodischaltglied derart aufgebaut sein, daß es mechanisch oder steuertechnisch verhindert, daß das Steuerglied in eine Schaltstellung gebracht wird, in welcher mit der Antriebseinheit eine Maximalkraft zum Betätigen des beweglichen Werkzeugs erzeugt werden kann.

Die nachfolgende Beschreibung einer bevorzugten Ausführungsform der Erfindung dient im Zusammenhang mit der Zeichnung der näheren Erläuterung. Es zeigen:
- Figur 1:: eine Längsschnittansicht durch eine Knochenstanze in einer unbetätigten Stellung;
- Figur 2:: eine vergrößerte Ansicht des Griffteils des Instruments in Figur 1;
- Figur 3:: eine ausschnittsweise Vergrößerung eines in Figur 2 dargestellten Schaltventils;
- Figur 4:: eine Ansicht ähnlich Figur 2 des Instruments in einer ersten Arbeitsstellung;
- Figur 5:: eine Ansicht ähnlich Figur 3, jedoch in der ersten Arbeitsstellung;
- Figur 6:: eine Ansicht ähnlich Figur 2 in einer zweiten Arbeitsstellung; und
- Figur 7:: eine Ansicht ähnlich Figur 3, jedoch in der zweiten Arbeitsstellung.

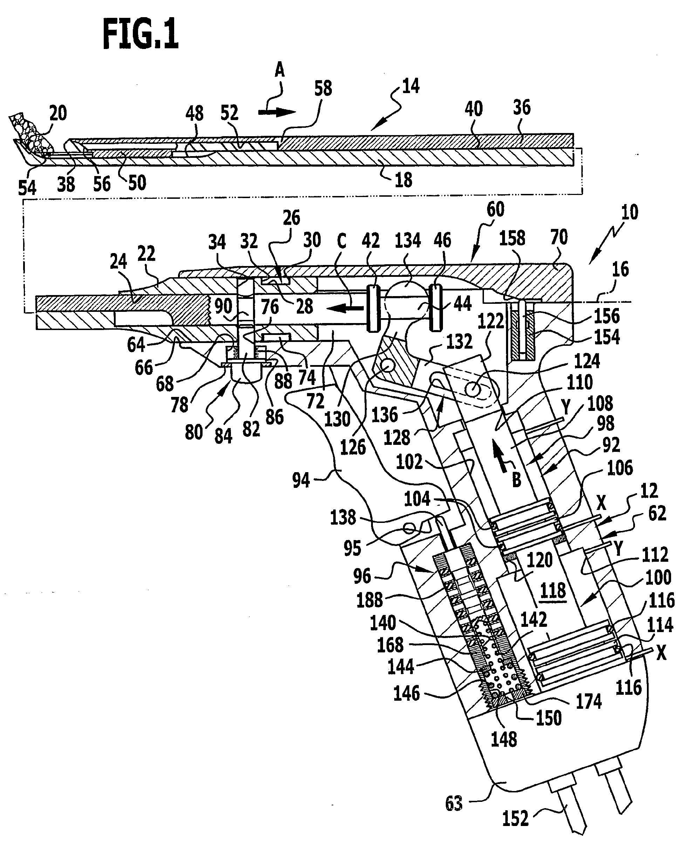

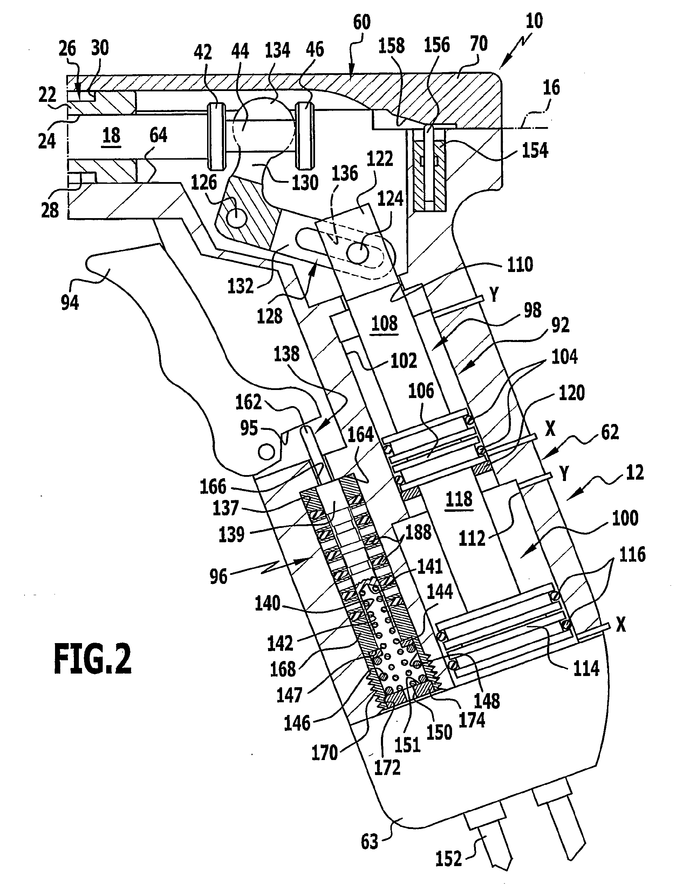

In den Figuren 1 bis 7 ist ein erfindungsgemäßes chirurgisches Instrument in Form einer Knochenstanze dargestellt, die insgesamt mit dem Bezugszeichen 10 versehen ist. Die Knochenstanze 10 umfaßt zwei wesentliche Baugruppen, nämlich ein Griffteil 12 sowie ein mit dem Bezugszeichen 14 versehenes Stanzwerkzeug.

Das Stanzwerkzeug 14 umfaßt einen sich in einer Längsrichtung 16 erstreckenden langgestreckten Schaft 18, welcher an seinem distalen Ende eine um etwa 45° gegenüber der Längsrichtung 16 geneigte Schneidplatte 20 trägt. Ein proximales Ende des Schafts bildet ein Kupplungsstück 22, welches in Form eines im Querschnitt quadratischen, langgestreckten Quaders mit angefasten Längskanten ausgebildet ist. Das Kupplungsstück 22 ist mit einer die Längsachse 16 definierenden Durchgangsbohrung 24 versehen. Des weiteren ist etwas beabstandet vom proximalen Ende des Schafts 18 am Kupplungstück 22 eine sich in Umfangsrichtung erstreckende nutartige Vertiefung 26 ausgebildet, wobei ein Boden der Vertiefung einen Vierkant 28 bildet. Er dient als erstes Kupplungselement zum verdrehsicheren Verbinden des Stanzwerkzeugs 14 mit dem Griffteil 12. Eine sich proximalseitig an den Vierkant 28 anschließende Seitenwand 30 der Vertiefung 26 bildet einen in distaler Richtung wirkenden Anschlag. Eine sich distalseitig an die Vertiefung 26 anschließende Seitenwand 32 bildet einen in proximaler Richtung wirkenden Anschlag.

Distalseitig der Vertiefung 26 sind am Kupplungsstück 22 vier identische Sicherungsbohrungen 34 ausgebildet, die sich in radialer Richtung bezogen auf die Längsachse 16 bis zur Durchgangsbohrung 24 durch das Kupplungsstück 22 hindurch erstrecken. Ferner verjüngt sich das quaderförmige Kupplungsstück 22 distalseitig im Durchmesser und weist an seinem Ende eine im wesentlichen rundhülsenartige Form auf.

Das Stanzwerkzeug 14 umfaßt eine Stanze 36, welche an ihrem distalen Ende eine gegenüber der Längsachse 16 um denselben Winkel wie die Schneidplatte 20 geneigte Schneide 38 trägt. Die Stanze 38 liegt im wesentlichen auf ihrer gesamten Länge flächig an einer Schaftfläche 40 des Schafts 18 an. Proximalseitig durchsetzt die Stanze 36 die Durchgangsbohrung 24 des Kupplungsstücks 22. In diesem Bereich ist die distalseitig im übrigen im Querschnitt im wesentlichen quaderförmig ausgebildete Stanze 36 zylindrisch geformt. An den zylindrischen Abschnitt der Stanze 36 schließt sich proximalseitig ein quadratischer, plattenförmiger Flansch 42 an, welcher distalseitig einen Kupplungsvierkant 44 begrenzt und einen in proximaler Richtung wirkenden Anschlag bildet.

An den Kupplungsvierkant 44 schließt sich, ein Ende der Stanze 36 bildend, eine quadratische Abschlußplatte 46 an, die einen in distaler Richtung wirkenden Anschlag bildet. Der Flansch 42 bildet zudem einen in distaler Richtung wirkenden Anschlag, welcher am proximalen Ende des Kupplungsstücks 22 anschlägt, wenn die Schneide 38 ihre distalste Stellung einnimmt, also an der Schneidplatte 20 anliegt.

Zur Stabilisierung einer Bewegung der Stanze 36 relativ zum Schaft 18 ist zusätzlich zu der durch das Kupplungsstück 22 ausgebildeten Führung am distalen Ende Schafts eine Führungsnut 48 angeordnet, welche sich von der Schaftfläche 40 weg im Querschnitt erweitert. In der Führungsnut 48 ist ein an der Stanze 36 abstehender Führungsvorsprung 50 geführt, welcher sich parallel zur Längsachse 16 ausgehend vom distalen Ende der Stanze 36 in proximaler Richtung erstreckt. Der Führungsvorsprung 50 und die Führungsnut 48 sind im wesentlichen korrespondierend ausgebildet, beispielsweise können sie eine Schwalbenschwanzform aufweisen.

Die Stanze 36 ist ausgehend von der Schneide 38 mit einer sacklochartigen, sich parallel zur Längsachse 16 erstreckenden Ausnehmung versehen, die als Gewebespeicher 52 für mit der Knochenstanze 10 entferntes Gewebe dient. Mit der Schneide ausgestanztes Gewebe 54 wird durch eine distalseitige Öffnung 56 des Gewebespeichers 52 in diesen hineingeschoben und bei weiterem Ausstanzen von Gewebe 54 in proximaler Richtung vorgeschoben, das heißt in Richtung des Pfeils A in Figur 1. Bei dem in den Figuren dargestellten Ausführungsbeispiel ist zum Entleeren des Gewebespeichers 52 an der Stanze 36 eine Entleerungsöffnung 58 vorgesehen, welche durch eine schräg zur Längsachse verlaufende Bohrung der Stanze 16 gebildet wird, welche eine Fluidverbindung zwischen dem Gewebespeicher 52 und einer Umgebung des Stanzwerkzeugs 14 herstellt. Ausgestanztes und im Gewebespeicher 52 gespeichertes Gewebe 54 kann durch die Entleerungsöffnung 58 nach außen abgegeben werden. Bei einer alternativen, nicht dargestellten Ausführungsform der Erfindung kann in der Stanze 36 ein Auswerfer in Form eines parallel zur Längsachse 16 verschieblichen Stabes vorgesehen sein, mit welchem ausgehend vom proximalen Ende des Gewebespeichers 52 in diesem gespeichertes Gewebe 54 in distaler Richtung durch die Öffnung 56 herausgeschoben werden kann.

Der Griffteil 12 der Knochenstanze 10 ist im wesentlichen in Form eines einem Pistolengriff ähnlichen Gehäuses ausgebildet. Er umfaßt einen sich parallel zur Längsachse 16 erstreckenden, langgestreckten quaderförmigen oberen Gehäuseteil 60, welcher eine in distaler Richtung weisende Öffnung 66 aufweist und einen im wesentlichen langgestreckten, quaderförmigen Aufnahmeraum 64 für das Kupplungsstück 22 des Schafts 18 bildet. Quer dazu und leicht in proximaler Richtung geneigt steht vom oberen Gehäuseteil 60 ein Griff 62 ab.

Der obere Gehäuseteil 60 umfaßt einen Schiebedeckel 70, welcher parallel zur Längsachse 16 mittels zweier in Form rippenartiger Vorsprünge ausgebildeter Führungen beidseitig in Längsnuten an sich parallel zur Längsachse 16 erstreckenden Seitenwänden 72 des oberen Gehäuseteils 60 geführt ist. In seiner distalsten Stellung bedeckt der Schiebedeckel 70 den Aufnahmeraum 64 vollständig, in seiner proximalsten Stellung gibt er den Aufnahmeraum 64 überwiegend frei, so daß eine Einlegeöffnung gebildet wird, durch die das Kupplungsstück 22 in den Aufnahmeraum 64 in einer Richtung quer zur Längsachse 16 in den Aufnahmeraum 64 eingelegt werden kann.

Auf Innenseiten der Seitenwände 72 stehen einander gegenüberliegend zwei aufeinander zu weisende, ein zweites Kupplungselement bildende Kupplungsvorsprünge 74 ab. Sie sind im wesentlichen in Form flacher Quader ausgebildet, deren Abmessungen so gewählt sind, daß die beiden Kupplungsvorsprünge 74 zwischen die Seitenwände 30 und 32 in die Vertiefung 26 eintauchen können und im wesentlichen am Vierkant 28 anliegen. Dadurch wird das Stanzwerkzeug 14 axial am Griffteil 12 festgelegt.

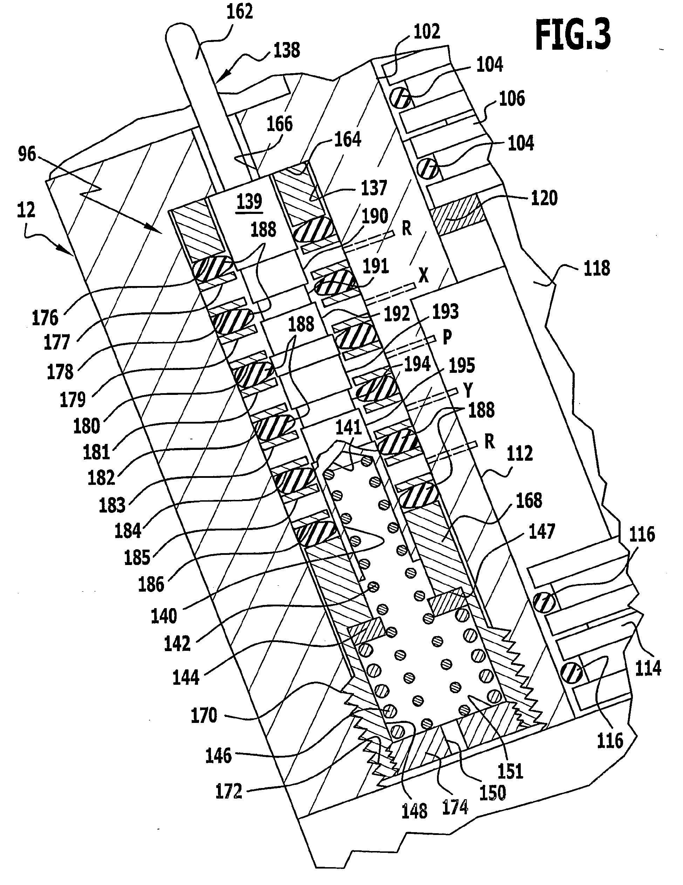

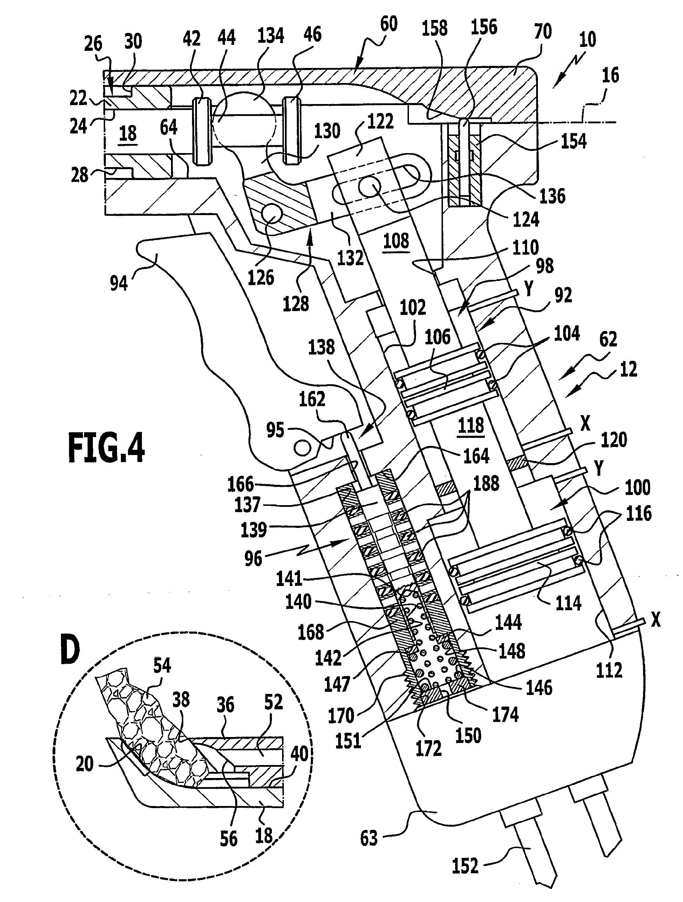

Wie besonders gut in Figur 1 zu erkennen, ist distalseitig der Kupplungsvorsprünge 74 der obere Gehäuseteil 60 auf seiner Unterseite mit einer nach unten geöffneten topfförmigen Vertiefung 68 versehen, die zum Aufnahmeraum 64 hin durch eine Bohrung 76 verbunden ist. Die Vertiefung 68 ist vom Aufnahmeraum 64 weg weisend von außen her mit einer Scheibe 78 versehen, welche einen radial nach innen vorstehenden Flansch bildet. Die Vertiefung 68 dient zur Aufnahme eines Sicherungsknopfes 80, welcher einen die Bohrung 76 durchsetzenden zylindrischen Bolzen 82 umfaßt, der einen Kopf 84 trägt, wobei im Übergangsbereich zwischen dem Kopf 84 und dem Bolzen 82 ein radial nach außen stehender Ringflansch 86 ausgebildet ist. Benachbart der Bohrung 76 stützt sich in der Vertiefung, den Bolzen 82 umgebend, eine Schraubenfeder 88 ab, welche mit Ihrem anderen Ende an der Unterseite des Kopfes 84 anliegt. Dadurch wird der Ringflansch 86 in einer Grund- oder Ruhestellung, wie sie in den Figuren 1 und 3 dargestellt ist, gegen die Scheibe 78 gedrückt. Gegen die Wirkung der Schraubenfeder 88 kann der Sicherungsknopf 80 in Richtung auf den Aufnahmeraum 64 hin bewegt werden. Der Sicherungsknopf 80 ist zudem derart angeordnet, daß er bei in den Aufnahmeraum 64 eingelegtem Kupplungsstück 22 in der Grundstellung, in welcher die Schraubenfeder 88 den Ringflansch 86 gegen die Scheibe 78 drückt, die Sicherungsbohrung 34 im wesentlichen formschlüssig ausfüllt. Der Sicherungsknopf 80 kann bei eingelegtem Kupplungsstück 22 jedoch nur in Richtung auf den Aufnahmeraum 64 hin bewegt werden, wenn eine den zylindrischen Teil des die Durchgangsbohrung 24 durchsetzenden Stanzwerkzeugs 14 in Umfangsrichtung umgebende Ringnut 90 mit den Sicherungsbohrungen 34 in radialer Richtung überlappt. Dies ist, wie in Figur 1 dargestellt, dann der Fall, wenn das Stanzwerkzeug 14 relativ zum Schaft 18 seine proximalste Stellung einnimmt, das heißt, wenn der Abstand zwischen der Schneide 38 und der Schneidplatte 20 maximal ist. Wird der Sicherungsknopf 80 dann gedrückt, so taucht ein vorderes Ende des Bolzens 82, wie in Figur 4 dargestellt, in die Ringnut 90 ein, wodurch eine Bewegung des Stanzwerkzeugs 14 parallel zur Längsachse 16 verhindert wird.

Eine insgesamt mit dem Bezugszeichen 92 versehene pneumatische Antriebsvorrichtung ist im wesentlichen im beziehungsweise am Griff 62 angeordnet. Die Antriebsvorrichtung 92 umfaßt einen auf einer in distaler Richtung weisenden Seite des Griffs 62, direkt unterhalb des oberen Gehäuseteils 60 angeordneten und um eine quer zur Längsachse 16 schwenkbar gelagerten Betätigungshebel 94, ein mit dem Betätigungshebel 94 betätigbares Schaltventil 96 sowie eine Antriebseinheit, welche einen ersten Pneumatikzylinder 98 und eine zweiten Pneumatikzylinder 100 umfaßt.

Die beiden Pneumatikzylinder 98 und 100 sind jeweils doppeltwirkend ausgebildet, wobei der erste Pneumatikzylinder 98 einen zylindrischen Kolbenraum 102 umfaßt, in welchem ein mittels zwei Dichtringen 104 abgedichteter Kolben 106 parallel zur Symmetrieachse des Kolbenraums 102 verschiebbar ist. Der Kolbenraum 102 ist in gleicher Weise wie der Griff 62 etwas gegenüber der Längsachse 16 geneigt. Vom Kolben 106 steht eine Kolbenstange 108 in Richtung auf den Aufnahmeraum 64 hin weisend ab, welche in einer Kolbenbohrung 110 geführt ist, die den Kolbenraum 102 mit dem Aufnahmeraum 64 verbindet. Ein Kolbenraum 112 schließt sich direkt an den Kolbenraum 102 an, und zwar mit einem etwas größeren Innendurchmesser. In diesem ist ein verschieblich gelagerter Kolben 114 mittels zweier Dichtringe 116 abgedichtet und über eine Kolbenstange 118 direkt mit dem Kolben 106 verbunden. Die beiden Pneumatikzylinder 98 und 100 bilden so eine insgesamt rotationssymmetrische Kolben-Zylinder-Einheit. Die Kolbenräume 102 und 112 sind durch eine Dichtscheibe 120 getrennt, die von der Kolbenstange 118 durchsetzt wird. Jeder der beiden Pneumatikzylinder 98 und 100 ist mit jeweils zwei Anschlüssen X und Y versehen, durch die die Kolben 106 und 114 mit Druckluft beaufschlagt werden können. Die Anschlüsse X sind dabei jeweils an einem vom Aufnahmeraum 64 wegweisenden Ende des jeweiligen Kolbenraums 102 beziehungsweise 112 angeordnet, so daß die Kolben 106 und 114 bei Druckluftbeaufschlagung durch die Anschlüsse X in Richtung des Pfeils B in Figur 1 bewegt werden. Die Anschlüsse Y sind jeweils am anderen Ende der beiden Kolbenräume 102 und 112 angeordnet, so daß durch Druckluftbeaufschlagung der Kolben 106 und 114 über die Anschlüsse Y die Kolben 106 und 114 in einer dem Pfeil B entgegengesetzten Richtung bewegt werden.

An die Kolbenstange 108 schließt sich in Richtung auf den Aufnahmeraum 64 hin weisend eine quaderförmige Verlängerung 122 an, welche einen quer zur Längsachse 16 und zur Längsachse der Kolbenstange 108 beidseitig vorstehenden Antriebsstift 124 trägt. Parallel zum Antriebsstift 124 verlaufend ist im Übergangsbereich des oberen Gehäuseteils 60 zum Griff 62 eine Lagerwelle 126 angeordnet, die zur Lagerung eines L-förmigen Winkelhebels 128 dient. Ein erster Hebelarm 130 des Winkelhebels 128 weist im wesentlichen in Richtung auf den Aufnahmeraum 64 hin, ein zweiter Hebelarm 132 ist im wesentlichen parallel zum Aufnahmeraum 64 in proximaler Richtung ausgerichtet.

Freie Enden der beiden Hebelarme 130 und 132 sind jeweils in Richtung auf die Lagerwelle 126 parallel zu einer Symmetrieebene der Knochenstanze 10 geschlitzt, so daß die freien Enden jeweils U-förmig ausgebildet sind. Der Winkelhebel 128 ist derart angeordnet, daß das geschlitzte freie Ende des ersten Hebelarms 130, welches in Form zweier scheibenförmiger Mitnehmer 134 ausgebildet ist, den Kupplungsvierkant 44 zwischen dem Flansch 42 und der Abschlußplatte 46 beidseitig umgreift, seitlich aber nicht über den Flansch 42 und die Abschlußplatte 46 vorsteht. Der erste Hebelarm 130 ist zudem derart gegenüber den Mitnehmern 134 verjüngt, daß nur diese in Anlage mit dem Flansch 42 und der Abschlußplatte 46 kommen können. Das geschlitzte Ende des zweiten Hebelarms 132 ist mit jeweils einem langlochartigen Schlitz 136 versehen und umgibt die Verlängerung 122, wobei der Antriebsstift 124 in die Schlitze 136 eintaucht und in diesem geführt ist.

Der zweite Hebelarm 132 ist etwa doppelt so lang wie der erste Hebelarm 130. Somit bildet der Winkelhebel 128 einerseits eine Kraftumlenkeinheit einer Antriebskraft, welche in Richtung des Pfeils B in Figur 1 von dem Pneumatikzylinder 98 und 100 erzeugt werden kann, in eine parallel zur Längsachse 16 verlaufende Antriebsrichtung, die durch den Pfeil C in Figur 1 symbolisiert wird. Gleichzeitig bildet der Winkelhebel 128 auch eine Übersetzungseinheit, mit welcher die von der Kolben-Zylinder-Einheit erzeugte Antriebskraft aufgrund des Längenverhältnisses der Hebelarme 130 und 132 verdoppelt wird.

Zur Steuerung der Pneumatikzylinder 98 und 100 dient das Schaltventil 96, welches im Bereich eines vom Aufnahmeraum 64 weg weisenden Endes 63 des Griffs 62 in einem parallel zum zweiten Pneumatikzylinder 100 verlaufenden, langgestreckten zylindrischen Hohlraum 137 angeordnet ist und einen vom Betätigungselement 94 betätigbaren, parallel zu den Kolben 106 und 114 verschiebbaren Stößel 138 umfaßt. Das Schaltventil 96 ist insgesamt rotationssymmetrisch ausgebildet, wobei der Stößel 138 einen das Schaltventil 96 durchsetzenden, langgestreckten zylindrischen Stößelkörper 139 aufweist. Ein in Richtung auf den Betätigungshebel 94 weisendes Ende des Stößels 138 bildet eine einstufig im Durchmesser reduzierte Stößelspitze 162, welche eine den Hohlraum 137 an einer inneren Stirnseite 164 durchsetzende Bohrung 166 durchsetzt und direkt an einer auf das Ende 63 hin weisenden Betätigungsfläche 95 des Betätigungshebels 94 anliegt. Die Bohrung 166 ist im Durchmesser kleiner als ein Außendurchmesser des Stößelskörpers 139, so daß die eine innere Ringfläche bildende Stirnseite 164 einen Anschlag für den Stößelkörper 139 bildet.

Ein vom Betätigungselement 94 weg weisendes Ende des Stößelkörpers 139 ist mit einem in Richtung auf das Ende 63 hin geöffneten Sackloch 140 versehen, so daß dieses Ende des Stößelkörpers 137 hülsenartig ausgebildet ist. An einem in Richtung auf das Ende 63 hin weisenden Sacklochboden 141 des Sacklochs 140 stützt sich eine Schraubenfeder 142 ab.

Den Hohlraum 137 auf seiner gesamten Länge ausfüllend ist in diesen ein hülsenartiger Ventileinsatz 168 eingesetzt, welcher vom Stößelkörper 139 längs seiner Symmetrieachse durchsetzt wird. Der Ventileinsatz 168 ist benachbart seinem auf das Ende 63 hin weisenden Ende mit einem kurzen Außengewindeabschnitt 170 versehen, welcher korrespondierend zu einem kurzen Innengewindeabschnitt 172 benachbart einem auf das Ende 63 hin weisenden Ende des Hohlraums 137 ausgebildet ist. Der Ventileinsatz 168 ist mittels des Au-Bengewindeabschnitts 170 in den Hohlraum 137 eingeschraubt und so axial festgelegt.

Ein Innendurchmesser des Ventileinsatzes 168 entspricht in etwa einem maximalen Außendurchmesser des Stößelkörpers 139. Ferner ist der Ventileinsatz 168 im Innendurchmesser in etwa auf der Länge des Außengewindeabschnitts 170 im Innendurchmesser einstufig erweitert, so daß ein Ventilraum 148 ausgebildet ist, dessen Durchmesser etwas größer ist als ein maximaler Außendurchmesser des Stößelkörpers 139. Der Ventilraum 148 ist in Richtung auf das Ende 63 hin durch einen Deckel 174 verschlossen, dessen Innenseite einen in Richtung auf den Betätigungshebel 94 hin weisenden Boden 151 bildet.

Der Boden 151 ist zentral von einer Einlaßöffnung 150 in Form einer Bohrung durchbrochen. Durch die einstufige Erweiterung im Inneren des Ventileinsatzes 168 ist eine in Richtung auf den Boden 151 hin weisende, parallel zu diesem verlaufende Ringwand 147 ausgebildet. Diese bildet einen Anschlag für eine Scheibe 144, deren Außendurchmesser mit dem Innendurchmesser des Ventilraums 148 übereinstimmt und die eine kreisrunde Durchbrechung aufweist, die im Durchmesser etwas kleiner ist als ein maximaler Außendurchmesser des Stößelkörpers 139. Die bereits erwähnte Schraubenfeder 142 stützt sich mit ihrem anderen Ende, die Einlaßöffnung 150 des Ventilraums umgebend am Boden 151 ab. Ihr maximaler Außendurchmesser ist so gewählt, daß sie die Durchbrechung der Scheibe 144 spielfrei durchsetzt. Die Schraubenfeder 142 drückt den Stößel 138 in Richtung auf den Betätigungshebel 94 hin, so daß das Schaltventil 96 im unbetätigten Zustand mit seiner Stößelspitze 162 an der Betätigungsfläche 95 des Betätigungshebels 94 anliegt. Eine weitere Schraubenfeder 146 stützt sich einerseits an der Scheibe 144, andererseits am Boden 151 ab und umgibt die Schraubenfeder 142. Die Schraubenfeder 146 weist eine Federkonstante auf, die die der Schraubenfeder 142 um ein mehrfaches übersteigt.

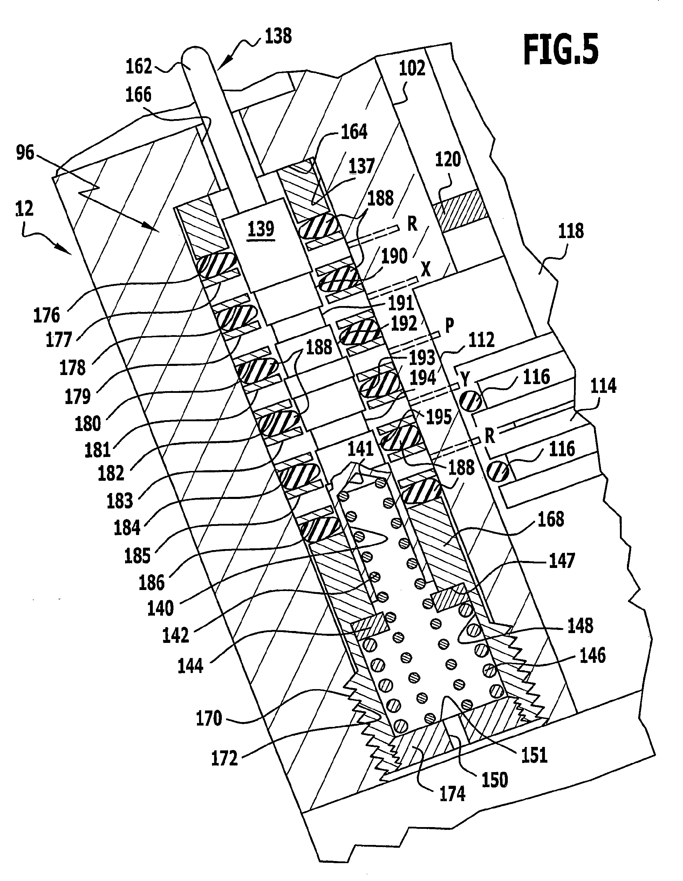

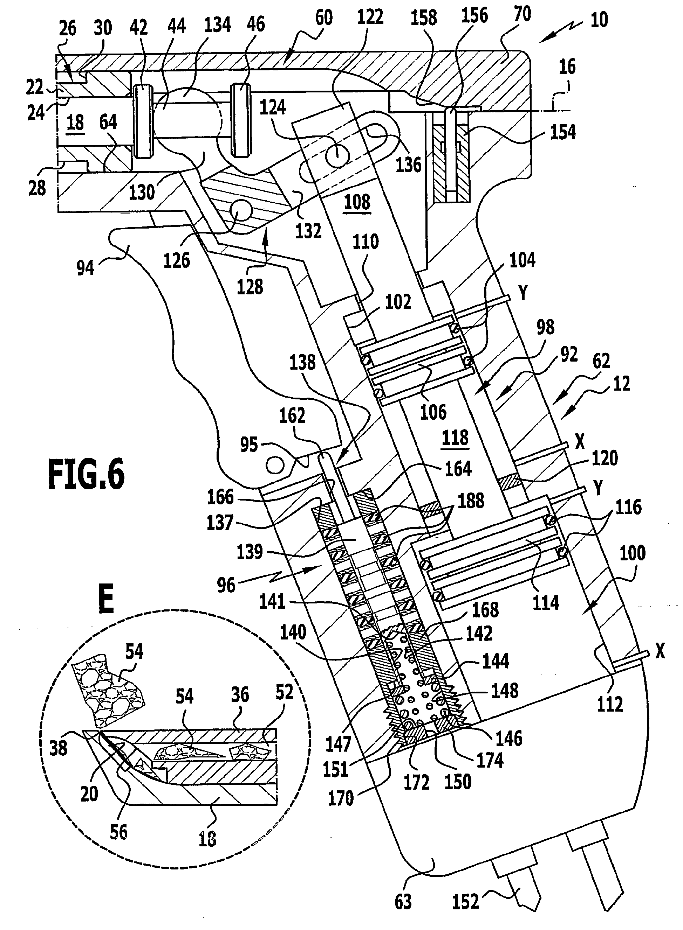

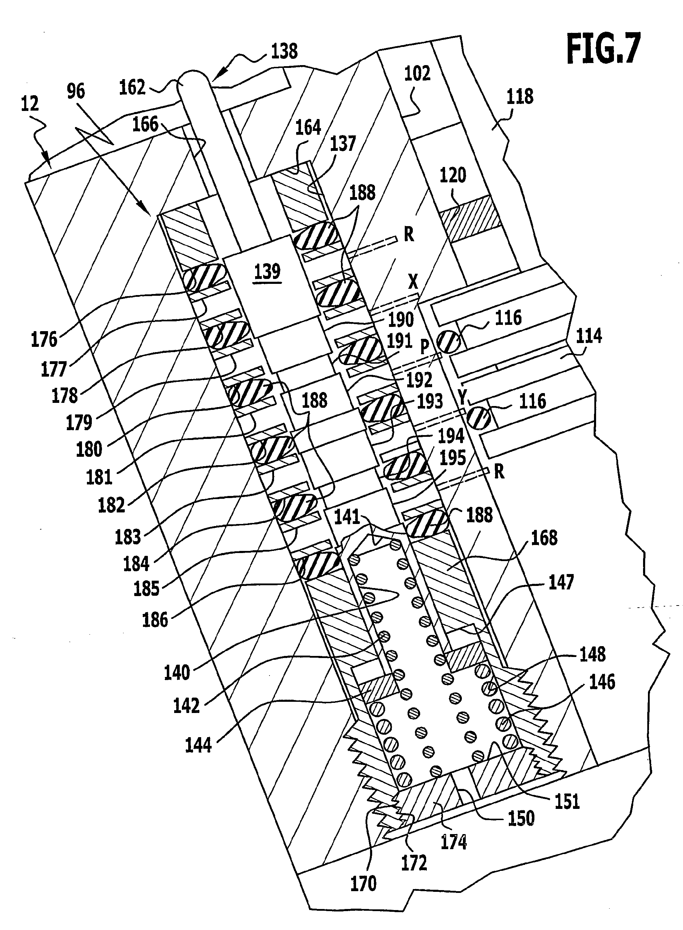

Der Stößel 138 kann von einer unbetätigten Stellung, wie sie in den Figuren 1 bis 3 dargestellt ist, entgegen der Wirkung der Schraubenfeder 142 in Richtung auf die Scheibe 144 hin bewegt werden, bis das hülsenartige Ende des Stößelkörpers 139 an der Scheibe 144 anschlägt. Das Schaltventil 96 nimmt dann eine erste Schaltstellung ein, wie sie in den Figuren 4 und 5 dargestellt ist. Wird der Betätigungshebel 94 weiter verschwenkt, dann nimmt das hülsenartige Ende des Stößelkörpers 139 die Scheibe 144 nun auch entgegen der Wirkung der Schraubenfeder 146 mit. Diese zweite Schaltstellung ist in den Figuren 6 und 7 dargestellt. So kann bei Betätigung des Betätigungshebels 94 von einer Bedienperson aufgrund der durch die Schraubenfedern 142 und 146 erzeugten Rückstellkräfte auf den Stößel 138 auf eine Schaltstellung des Schaltventils 96 geschlossen werden. Da die Federkonstante der Schraubenfeder 146 um ein vielfaches größer ist als die der Schraubenfeder 142, erhält eine Bedienperson eine taktile Rückmeldung, daß von der ersten Schaltstellung in die zweite Schaltstellung gewechselt wurde.

Der Ventileinsatz 168 ist ausgehend von seinem an der Stirnseite 164 anliegenden Ende mit insgesamt elf identischen Ringnuten 176 bis 186 versehen, wobei in allen geradzahligen Ringnuten 176, 178, 180, 182, 184, 186 jeweils ein eine Dichtlippe für den Stößelkörper 139 bildender Dichtring 188 eingesetzt ist.

Der Stößelkörper 139 ist mit einem Ringnutsystem versehen, welches anhand Figur 3 beschrieben wird. In der in Figur 3 dargestellten Grundstellung des Schaltventils 96 dichtet der in die Ringnut 176 eingelegte Dichtring 188 den Stößelkörper 139 in Richtung auf die Bohrung 166 ab. Auf Höhe der Ringnut 177 ist ein Außendurchmesser des Stößelkörpers 139 einstufig verringert und bildet eine Ringnut 190. Gegenüberliegend dem in der Ringnut 178 eingesetzten Dichtring 188 ist der Außendurchmesser des Stößelkörpers 139 nochmals einstufig verjüngert und bildet eine Ringnut 191. Danach nimmt der Außendurchmesser des Stößelkörpers 139 wieder einstufig zu und bildet eine der Ringnut 189 gegenüberliegende Ringnut 192. Der in die Ringnut 180 eingelegte Dichtring 188 liegt wieder dichtend am maximalen Außendurchmesser des Stößelkörpers 139 an. Daran benachbart schließt sich wieder eine Ringnut 193 am Stößelkörper 139 an, die im Außendurchmesser einstufig verjüngt ist, jedoch einen größeren Außendurchmesser aufweist als die identischen Ringnuten 190 und 192. Der in die Ringnut 182 eingelegten Dichtring 188 gegenüberliegend ist eine weitere Ringnut 194, deren Außendurchmesser in etwa dem der Ringnute 190 und 192 entspricht. Daran anschließend ist wiederum eine Ringnut 195 ausgebildet, die der Ringnut 193 entspricht und der Ringnut 184 gegenüberliegend ausgebildet ist. Der in die Ringnut 184 eingelegte Dichtring 188 dichtet wiederum den Stößelkörper 139 vollständig ab.

Die ungeradzahligen Ringnuten 177, 179, 181, 183 und 185 sind mit Lufteinlaßöffnungen X, Y und P versehen, die in den Figuren schematisch gestrichelt dargestellt und mit den beiden Pneumatikzylindern 98 und 100 sowie einer nicht dargestellten Druckluftquelle wie folgt verbunden sind. Die Ringnut 181 ist mit einer mit P bezeichneten Einlaßöffnung versehen, die mit der nicht dargestellten Druckluftquelle verbunden ist. Die an der Ringnut 179 vorgesehene Einlaßöffnung X ist mit den beiden Einlaßöffnungen der Pneumatikzylinder 98 und 100 verbunden, die dort ebenfalls mit X bezeichnet sind. Ferner ist die Ringnut 183 mit einer mit Y gekennzeichneten Einlaßöffnung versehen, welche wiederum mit den mit Y bezeichneten Einlaßöffnungen der Pneumatikzylinder 98 und 100 verbunden ist. Ferner sind mit den Ringnuten 177 und 185 Einlaßöffnungen verbunden, die beide mit R bezeichnet sind. Diese bilden Entlüftungsöffnungen, über die Druckluft aus dem System entweichen kann.

Die drei möglichen Schaltstellungen des Schaltventils 96 werden nachfolgend naher erläutert. In der in den Figuren 1 bis 3 dargestellten unbetätigten Stellung des Betätigungshebels 94, einer sogenannten Ruhe- oder Ausgangsstellung, stehen, wie bereits beschrieben die mit den Ringnuten 177 und 179 verbundenen Einlaßöffnungen X und R des Hohlraums 137 in Fuidverbindung. Dadurch werden die beiden Pneumatikzylinder 89 und 100 über ihre Einlaßöffnungen X entlüftet. Ebenso stehen die mit den Ringnuten 181 und 183 verbundenen Einlaßöffnungen P und Y durch die mittels der Ringnuten 193, 194 und 195 definierten Ringräume in Fluidverbindung. Auf diese Weise ist die Druckluftquelle über das Schaltventil 96 hin mit den Einlaßöffnungen Y der Pneumatikzylinder 98 und 100 derart verbunden, daß die beiden Pneumatikzylinder 98 und 100 gemeinsam entgegen der durch den Pfeil B angegebenen Richtung in ihre Endstellung bewegt werden. Das Instrument nimmt so eine Haltestellung ein, die der Ruhe- oder Grundstellung entspricht. Ein Abstand zwischen der Schneide 38 und der Schneidplatte 20 ist maximal.

Wird der Betätigungshebel 94 in Richtung auf den Griff 62 hin verschwenkt, so wird der Stößel 138 in Richtung auf die Scheibe 144 hin bewegt. Das Schaltventil 96 nimmt dann die in den Figuren 4 und 5 dargestellte erste Schaltstellung ein. Durch die unterschiedlich gewählten Außendurchmesser der Ringnuten 190 bis 195 sind einerseits die Ringnuten 181, 179 und 177 miteinander verbunden, so daß Druckluft über den Einlaß P des Schaltventil 96 und über den Einlaß X an der Ringnut 179 zu den Einlässen X der beiden Pneumatikzylinder 98 und 100 strömen kann, wobei diese jedoch über die Verbindung mittels der Ringnute 190, 191 und 192 auch mit der Entlüftungsöffnung R der Ringnut 177 verbunden sind. Anderseits steht der Einlaß P an der Ringnut 181 über die Ringnute 193, 194 und 195 mit der Einlaß Y an der Ringnut 183 und dem Einlaß R an der Ringnut 185 in Fluidverbindung. Dadurch kann Druckluft über die Einlässe Y in die Pneumatikzylinder 98 und 100 strömen. Durch die speziell gewählten Außendurchmesser der Ringnuten 190, 191, 192 sowie 193, 194 und 195 werden die Fluidströme in die Einlässe X und Y, mit welchen die beiden Pneumatikzylinder 98 und 100 beaufschlagt werden, so verteilt, daß eine in Richtung des Pfeils B wirkende resultierende Antriebskraft etwa 20 Prozent einer maximal mit den beiden Pneumatikzylindern 98 und 100 erzeugbaren Vortriebskraft entspricht. Diese Kraft reicht, wie im gestrichelt umrandeten Bereich D in Figur 4 dargestellt, nicht aus, um zu entfernendes Gewebe 54 zu durchtrennen, sondern allenfalls die Schneide 38 in Anlage mit dem Gewebe 54 zu bringen.

Wird der Betätigungshebel 94 jedoch weiter in Richtung auf den Griff 62 bis zum Anschlag hin bewegt, so wird der Stößel 138 in die zweite Schaltstellung überführt, die in den Figuren 6 und 7 dargestellt ist. Die in den Ringnuten 178 sowie 182 und 186 eingelegten Dichtringe 188 dichten den Stößelkörper 139 vollständig ab, so daß einerseits eine Fluidverbindung zwischen der mit der Ringnut 179 verbundenen Einlaßöffnung X mit der mit der Ringnut 181 verbundenen Einlaßöffnung P hergestellt wird, andererseits eine Fluidverbindung zwischen der mit der Ringnut 183 verbundenen Einlaßöffnung Y und der mit der Ringnut 185 verbundenen Einlaßöffnung R. In dieser Schaltstellung werden beide Pneumatikzylinder 98 und 100 über ihre Einlaßöffnungen X von der Druckluftquelle mit Druckluft beaufschlagt. Eine entgegengesetzte Druckluftbeaufschlagung ist nicht möglich, da die Einlaßöffnungen Y mit der mit der Ringnut 185 verbundenen Entlüftungsöffnung R verbunden und dadurch entlüftet werden. Beide Pneumatikzylinder 98 und 100 werden somit in Richtung des Pfeils B in Figur 1 vorgetrieben, wodurch eine maximale Antriebskraft erzeugt und auf die Stanze 36 übertragen werden kann.

Um eine taktile Rückmeldung für eine Bedienperson der Knochenstanze weiter zu verbessern, kann die mit der Ringnut 179 verbündene Einlaßöffnung X auch mit der Einlaßöffnung 150 des Ventilraums 148 verbunden werden. Werden nämlich in der zweiten Schaltstellung, wie sie in den Figuren 6 und 7 dargestellt ist, beide der Pneumatikzylinder 98 und 100 mit Druckluft maximalen Drucks beaufschlagt, so wirkt dieser Druck auch gleichgerichtet mit den beiden Schraubenfedern 142 und 146, wodurch ein Operateur eine erhöhte Betätigungskraft aufbringen muß, um die zweite Schaltstellung des Betätigungshebels 94 beizubehalten.

Die Funktionsweise einer leicht modifizierten Ausführungsform der Antriebsvorrichtung 92 des Instruments wird nachfolgend näher dargelegt.

In einer unbetätigten Stellung des Betätigungshebels 94 wird der erste Pneumatikzylinder 98 über seinen Einlaß Y derart mit Druckluft beaufschlagt, daß der Kolben 106 gegen die Dichtscheibe 120 bewegt wird. Die Knochenstanze nimmt dann ihre geöffnete Stellung ein, das heißt der Mitnehmer 134 hält die Abschlußplatte 46 der Stanze 36 in ihrer proximalsten Stellung. Der Abstand zwischen der Schneide 38 und der Schneidplatte 20 ist maximal.

Wird der Betätigungshebel 94 entgegen der Schraubenfeder 142 bewegt, ohne daß der Stößel 138 an der Scheibe 144 anschlägt, wird nur der Kolben 106 in Richtung des Pfeils B in Figur 1 mittels Druckluftbeaufschlagung durch den Einlaß X zwischen dem Kolben 106 und der Drehscheibe 120 bewegt. Ein Verhältnis der Wirkungsquerschnitte des ersten Pneumatikzylinders 98 und des zweiten Pneumatikzylinders 100 beträgt etwa 1 : 4, so daß zunächst nur eine Antriebskraft in Höhe von etwa 20 Prozent einer maximal möglichen Antriebskraft erzeugt und über den Winkelhebel 128 auf die Stanze 36 übertragen wird. Die so verminderte, auf die Stanze 36 wirkende Kraft reicht typischerweise nicht dazu aus, um zu entfernendes Gewebe 54, beispielsweise Knochenteile, zu durchtrennen. Die Kraft reicht, wie im gestrichelt umgrenzten Bereich D in Figur 4 dargestellt, nur dazu aus, die Schneide 38 an das Gewebe heranzuführen. In dieser ersten Betätigungsstellung des Betätigungshebels kann eine Bedienperson der Knochenstanze 10 damit die Schneidplatte 20 in gewünschter Weise an das zu entfernende Gewebe 54 anlegen, ohne daß es bereits zu einer Durchtrennung des Gewebes 54 kommt.

Ist die Schnittposition bestimmt, dann kann der Betätigungshebel 94 voll durchgedrückt werden. Der Stößel 138 wird nunmehr auch entgegen der Schraubenfeder 146 bewegt, was die Bedienperson durch die größere aufzubringende Betätigungskraft spürt, die erforderlich ist, um den Betätigungshebel 94 zu verschwenken. Das Schaltventil 96 nimmt dann eine Schaltstellung ein, in welcher sowohl der erste Pneumatikzylinder 98 als auch der zweite Pneumatikzylinder 100 über ihre Einlässe X mit Druckluft beaufschlagt werden, und zwar derart, daß beide Kolben 106 und 114 in Richtung des Pfeils B bewegt werden. Damit wird eine Maximalkraft der Antriebsvorrichtung 92 über den Winkelhebel 128 auf die Stanze 36 übertragen, welche geeignet ist, Gewebe 54 in gewünschter Weise zu durchtrennen. Nicht mit Druckluft beaufschlagte Einlässe Y der Pneumatikzylinder 98 und 100 sind in dieser Stellung jeweils belüftet.

Wird der Betätigungshebel 94 wieder entlastet, so drücken die Schraubenfedern 142 und 146 den Stößel 138 in seine Ausgangsstellung zurück, die Stanze 36 wird dann wieder durch Druckbeaufschlagung des Pneumatikzylinders 98 in einer zum Pfeil B entgegengesetzten Richtung beaufschlagt, wodurch die Stanze 36 von ihrer in den Figuren6 und 7 dargestellten Schneidstellung über ihre in Figuren 4 und 5 dargestellte Zwischenstellung in ihre in Figur 1 dargestellte Grundstellung, also ihre proximalste Stellung, zurücküberführt wird.

So viel zur Funktionsweise der leicht modifizierten Ausführungsform der Antriebsvorrichtung 92.

Um ein versehentliches Betätigen der Antriebsvorrichtung 92 durch Drücken des Betätigungshebels 94 durch eine Bedienperson auszuschließen, ist im proximalen Bereich des oberen Gehäuseteils 60 ein Sicherheitsventil 154 angeordnet. Das Sicherheitsventil 154 umfaßt einen quer zur Längsachse 16 verschiebbar gelagerten, gegen eine Innenseite des Deckels 70 federnd vorgespannten Ventilstößel 156. Ist der Deckel, wie in Figur 1 dargestellt, geschlossen, nimmt der Ventilstößel 156 eine Stellung ein, bei welcher er eine Fluidverbindung zwischen der Druckluftzufuhrleitung 152 und der Einlaßöffnung 150 des Schaltventils 96 herstellt. Wird der Deckel 70 jedoch geöffnet, gleitet das aus dem Sicherheitsventil 154 vorstehende Ende des Ventilstößels 156 an einer Anlaufschräge 158 auf der Innenseite des Deckels 70 entlang, wodurch der federnd vorgespannte Ventilstößel 156 weiter aus einem Körper des Sicherheitsventils 154 herausbewegt wird. Dadurch wechselt das Sicherheitsventil 154 seine Schaltstellung, es unterbricht nunmehr eine Verbindung zwischen der Druckluftzufuhrleitung 152 und der Einlaßöffnung 150 des Schaltventils 96, so daß eine Betätigung des Betätigungshebels 94 wirkungslos bleibt, da die Antriebsvorrichtung 92 somit von der Druckluftquelle getrennt ist. Erst dann, wenn der Schiebedeckel 70 wieder vollständig geschlossen ist, wird der Ventilstößel 156 so weit in den Körper des Sicherheitsventils 154 hineingedrückt, daß das Sicherheitsventil 154 wieder die Schaltstellung einnimmt, in welcher die Druckluftzufuhrleitung 152 in Fluidverbindung mit der Einlaßöffnung 150 des Schaltventils 96 steht.

Muß die Knochenstanze 10 während eines Einsatzes gereinigt werden, beispielsweise weil sich Gewebe 54 zwischen der Schneide 38 und der Schneidplatte 20 verklemmt hat, muß ein Operateur die Knochenstanze 10 nicht aus der Hand legen. Eine dem Operateur assistierende Person kann einen Zwischenraum zwischen der Schneide 38 und der Schneidplatte 20 reinigen, ohne Gefahr zu laufen, verletzt zu werden, selbst dann, wenn der Operateur den Betätigungshebel 94 betätigt. In einer unbetätigten Stellung des Betätigungshebels 94 überlappen die Ringnut 90 und die Sicherungsbohrungen 34.

Dies ist in Figur 1 dargestellt. Die dem Operateur assistierende Person kann nun den Sicherungsknopf 80 entgegen der Wirkung der Schraubenfeder 88 drücken, so daß der Bolzen 82 in die Ringnut 90 eintaucht. Eine Bewegung der Stanze 36 in distaler Richtung wird dadurch verhindert, selbst dann, wenn der Operateur den Betätigungshebel 94 drücken würde. Ist das zu entfernende Gewebeteil 54 abgeschnitten entfernt, kann die dem Operateur assistierende Person den Sicherungsknopf 80 wieder loslassen und der Operateur kann den chirurgischen Eingriff fortsetzen. Der beschriebene Reinigungsvorgang läßt sich besonders leicht durchführen, da der Sicherungsknopf 80 weit genug entfernt vom Betätigunshebel 94 angeordnet ist, insbesondere distalseitig desselben, so daß der Sicherungsknopf 80 für die dem Operateur assistierende Person stets frei zugänglich ist.

Durch die besondere Ausgestaltung des Kupplungsstücks 22, insbesondere durch Vorsehen des Vierkants 28, ist es möglich, das Stanzwerkzeug 14 in vier unterschiedlichen Stellungen mit dem Griffteil 12 zu verbinden. In den Figuren 1 bis 5 ist das Stanzwerkzeug 14 mit dem Griffteil 12 derart verbunden, daß ein Zwischenraum zwischen der Schneidplatte 20 und der Schneide 38 nach oben weist. Die beiden Teile der Knochenstanze 10 können jedoch auch so miteinander verbunden werden, daß der Zwischenraum nach unten oder nach einer der beiden Seiten der Knochenstanze 10 weist. Zum Ändern einer Relativposition des Stanzwerkzeugs 14 zum Griffteil 12 muß lediglich der Schiebedeckel 70 in proximaler Richtung verschoben werden, bis der Aufnahmeraum 63 geöffnet und das Stanzwerkzeug 14 entnommen werden kann. Das Stanzwerkzeug 14 kann so um 90°, 180° oder 270° gedreht und wieder in den Aufnahmeraum 64 eingesetzt werden. Verschieben des Deckels 70 in distaler Richtung verschließt den Aufnahmeraum 64 und sichert zudem das Kupplungsstück 22 und damit das Stanzwerkzeug 14 am Griffteil 12.

## Patentansprüche

1. Chirurgisches Instrument (10) umfassend einen Handhabungsteil (12) und einen Werkzeugteil (14), wobei das Werkzeugteil (14) mindestens ein beweglich gelagertes Werkzeug (36) umfaßt, welches über einen Kraftübertragungs- und/oder Betätigungsmechanismus (92, 128), welcher vom Handhabungsteil (12) aus bedienbar ist, betätigbar ist, und daß der Kraftübertragungs- und/oder Betätigungsmechanismus (92, 128) eine mit einem Fluid betreibbare Antriebseinheit (92) umfaßt, wobei mit der Antriebseinheit (92) in einer ersten Antriebsstellung das beweglich gelagerte Werkzeug (36) mit einer ersten Betätigungskraft beaufschlagbar und in mindestens einer zweiten Antriebsstellung mit mindestens einer zweiten Betätigungskraft beaufschlagbar ist und wobei die Antriebseinheit (92) einen Linearantrieb umfaßt, wobei der Linearantrieb (92) zwei gekoppelte, Fluidzylinder (98, 100) umfaßt, **dadurch gekennzeichnet daß** die Fluidzylinder getrennt ansteuerbar sind.

2. Instrument nach Anspruch 1, **dadurch gekennzeichnet, daß** mit der Antriebseinheit (92) in einer dritten Betätigungsstellung das beweglich gelagerte Werkzeug (36) mit einer dritten Betätigungskraft beaufschlagbar ist.

3. Instrument nach Anspruch 2, **dadurch gekennzeichnet, daß** die dritte Betätigungskraft der Summe der ersten und zweiten Betätigungskraft entspricht.

4. Instrument nach einem der voranstehenden Ansprüche, **dadurch gekennzeichnet, daß** ein Verhältnis der ersten und zweiten Betätigungskraft im Bereich von 4:1 bis 9:1 liegt.

5. Instrument nach einem der voranstehenden Ansprüche, **dadurch gekennzeichnet, daß** die beiden Fluidzylinder (98, 100) unterschiedliche Wirkungsquerschnitte aufweisen.

6. Instrument nach Anspruch 5, **dadurch gekennzeichnet, daß** ein Verhältnis der Wirkungsquerschnitte in einem Bereich von 4:1 bis 9:1 liegt.

7. Instrument nach einem der voranstehenden Ansprüche, **dadurch gekennzeichnet, daß** die zwei Fluidzylinder (98, 100) Pneumatik- und/oder Hydraulikzylinder sind.

8. Instrument nach einem der voranstehenden Ansprüche, **dadurch gekennzeichnet, daß** das Instrument (10) ausgebildet ist zum Entfernen von Knochen, Knorpel oder derartigem Gewebe (54), mit einem sich in einer Längsrichtung (16) erstreckenden Schaft (18), welcher an seinem distalen Ende eine quer zur Längsrichtung (16) angeordnete oder relativ zu dieser geneigte Schneidplatte (20) trägt, daß das beweglich gelagerte Werkzeug ein am Schaft (18) verschiebbar gelagertes Schneidelement (36) ist, welches an seinem distalen Ende eine in Richtung auf die Schneidplatte (20) weisende Schneide (38) trägt, die an die Schneidplatte (20) heranführbar ist zum Schneiden von Gewebe (54).

9. Instrument nach einem der voranstehenden Ansprüche, **dadurch gekennzeichnet, daß** in einer Ruhestellung des Instruments (10), in welcher mit dem Kraftübertragungs- und/oder Betätigungsmechanismus (92, 128) keine Betätigungskraft in einer Betätigungsrichtung auf das Werkzeug (36) ausgeübt wird, mit dem Kraftübertragungs- und/oder Betätigungsmechanismus (92, 128) in einer der Betätigungsrichtung entgegengesetzten Halterichtung eine Haltekraft auf das Werkzeug (36) ausübbar ist.

10. Instrument nach Anspruch 9, **dadurch gekennzeichnet, daß** der Kraftübertragungs- und/oder Betätigungsmechanismus (92, 128) ein Betätigungsglied (94) umfaßt zum Betätigen der Antriebseinheit (92), daß das Betätigungsglied (94) von einer unbetätigten Ausgangsstellung, in welcher das Instrument (10) die Ruhestellung einnimmt, in eine erste Aktivierungsstellung bringbar ist, in welcher die Antriebseinheit (92) die erste Antriebsstellung einnimmt, und in mindestens eine zweite Aktivierungsstellung bringbar ist, in welcher die Antriebseinheit die zweite Antriebsstellung einnimmt.

11. Instrument nach Anspruch 10, **dadurch gekennzeichnet, daß** das Betätigungsglied (94) federnd vorgespannt in der Ausgangsstellung gehalten ist.

12. Instrument nach einem der Ansprüche 10 oder 11, **dadurch gekennzeichnet, daß** das Betätigungsglied (94) schwenkbar gelagert ist.

13. Instrument nach einem der Ansprüche 10 bis 12, **dadurch gekennzeichnet, daß** in der ersten Aktivierungsstellung eine erste Rückstellkraft auf das Betätigungsglied (94) wirkt, daß in der zweiten Aktivierungsstellung eine zweite Rückstellkraft wirkt und daß die zweite Rückstellkraft größer als die erste Rückstellkraft ist.

14. Instrument nach Anspruch 13, **dadurch gekennzeichnet, daß** die zweite Rückstellkraft mindestens doppelt so groß wie die erste Rückstellkraft ist.

15. Instrument nach einem der Ansprüche 13 oder 14, **dadurch gekennzeichnet, daß** zum Erzeugen der ersten und zweiten Rückstellkraft eine Rückstelleinheit (142, 146) umfassend ein erstes elastisches Rückstellglied (142) und ein zweites elastisches Rückstellglied (146) vorgesehen ist.

16. Instrument nach Anspruch 15, **dadurch gekennzeichnet, daß** das erste und/oder das zweite Rückstellglied ein Federelement (142, 146) ist.

17. Instrument nach einem der Ansprüche 15 oder 16, **dadurch gekennzeichnet, daß** die Rückstelleinheit (142, 146) direkt oder indirekt auf ein Steuerglied (96) zum Ansteuern der Antriebseinheit (92) oder auf das Betätigungsglied (94) wirkend ausgebildet ist.

18. Instrument nach einem der Ansprüche 10 bis 17, **dadurch gekennzeichnet, daß** ein Steuerglied (96) zum Ansteuern der Antriebseinheit (92) vorgesehen ist und daß das Betätigungsglied (94) direkt oder indirekt auf das Steuerglied (96) wirkt.

19. Instrument nach Anspruch 18, **dadurch gekennzeichnet, daß** das Steuerglied (96) mindestens ein Steuerventil umfaßt.

20. Instrument nach Anspruch 19, **dadurch gekennzeichnet, daß** das mindestens eine Steuerventil (96) mindestens drei unterschiedliche Schaltstellungen aufweist.

21. Instrument nach Anspruch 19 oder 20, **dadurch gekennzeichnet, daß** das Steuerventil (96) einen Ventilstößel (138) aufweist, daß am Ventilkörper (137) mindestens ein mit einer Fluidquelle verbindbarer Anschluß und für jeden doppeltwirkenden Fluidzylinder (98, 100) zwei Anschlüsse vorgesehen sind, daß in einer ersten Stellung des Ventilstößels (138) mindestens einer der Fluidzylinder (98, 100) mit Fluid in einer Halterichtung beaufschlagbar ist, daß in einer zweiten Stellung des Ventilstößels (138) mindestens einer der Fluidzylinder (98, 100) beidseitig mit unterschiedlichen Fluidströmen beaufschlagbar ist, daß in einer dritten Stellung des Ventilstößels (138) mindestens einer der Fluidzylinder (98, 100) entgegen der Halterichtung mit einem Fluid beaufschlagbar ist und daß in der zweiten Stellung des Ventilstößels (138) ein Fluidstrom auf die beiden Anschlüsse mindestens eines Fluidzylinders (98, 100) ungleich verteilt wird.

22. Instrument nach Anspruch 21, **dadurch gekennzeichnet, daß** der Ventilstößel (138) mit einer Mehrzahl von Ringnuten (190, 191, 192, 193, 194, 195) versehen ist und in einem Ventilkörper (137) in unterschiedlichen Schaltstellungen unterschiedliche Ringräume definiert, deren Querschnittsverhältnisse in Abhängigkeit eines Fluiddrucks derart aufeinander abgestimmt sind, daß in der zweiten Schaltstellung nur ein Bruchteil einer maximalen Betätigungskraft durch den mindestens einen Fluidzylinder (98, 100) erzeugbar ist.

23. Instrument nach einem der Ansprüche 19 bis 22, **dadurch gekennzeichnet, daß** mindestens zwei Steuerventile (96) vorgesehen sind mit jeweils mindestens zwei Schaltstellungen.

24. Instrument nach Anspruch 23, **dadurch gekennzeichnet, daß** die mindestens zwei Steuerventile (96) manuell getrennt betätigbar sind.

25. Instrument nach einem der voranstehenden Ansprüche, **dadurch gekennzeichnet, daß** am Werkzeugteil (14) mindestens eine Kodiereinheit vorgesehen ist zum Kodieren der Art und/oder des Typs des Werkzeugteils (14) und daß am Handhabungsteil (12) eine Dekodiereinheit vorgesehen ist zum Dekodieren der Art und/oder des Typs des Werkzeugteils (14).

26. Instrument nach Anspruch 25, **dadurch gekennzeichnet, daß** die mindestens eine Kodiereinheit keinen oder mindestens einen am Werkzeugteil (14) abstehenden Vorsprung oder mindestens eine am Werkzeugteil (14) angeordnete Ausnehmung umfaßt, daß die Dekodiereinheit ein zu dem mindestens einen Vorsprung oder der mindestens einen Ausnehmung korrespondierendes Betriebsmodischaltglied umfaßt, daß das Betriebsmodischaltglied mindestens eine erste und eine zweite Betriebsmodistellung aufweist und daß das Betriebsmodischaltglied entsprechend der Kodierung des Werkzeugteils (14) eine der mindestens zwei Betriebsmodistellungen einnimmt.

27. Instrument nach Anspruch 26, **dadurch gekennzeichnet, daß** die mindestens zwei Betriebsmodistellungen maximalen Betätigungskräften der Antriebseinheit (92) zugeordnet sind.

28. Instrument nach Anspruch 26 oder 27, **dadurch gekennzeichnet, daß** das Betriebsmodischaltglied mit dem Steuerglied (96) gekoppelt ist und daß das Betriebsmodischaltglied in mindestens einer Betriebsmodistellung mindestens eine Schaltstellung des Steuerglieds (96) direkt oder indirekt außer Kraft setzt.

## Claims

1. Surgical instrument (10) comprising a handling part (12) and a tool part (14), wherein the tool part (14) comprises at least one movably mounted tool (36), which is actuable by means of a force-transmitting and/or actuating mechanism (92, 128) that is operable from the handling part (12), and that the force-transmitting and/or actuating mechanism (92, 128) comprises a fluid-operable drive unit (92), wherein with the drive unit (92) in a first driving position the movably mounted tool (36) can be subjected to a first actuating force and in at least a second driving position can be subjected to at least a second actuating force and wherein the drive unit (92) comprises a linear drive, wherein the linear drive (92) comprises two coupled fluid cylinders (98, 100), **characterized in that** the fluid cylinders are separately controllable.

2. Instrument according to claim 1, **characterized in that** with the drive unit (92) in a third actuating position the movably mounted tool (36) can be subjected to a third actuating force.

3. Instrument according to claim 2, **characterized in that** the third actuating force corresponds to the sum of the first and second actuating force.

4. Instrument according to any one of the preceding claims, **characterized in that** a ratio of the first and second actuating force lies in the range of 4:1 to 9:1.

5. Instrument according to any one of the preceding claims, **characterized in that** the two fluid cylinders (98, 100) have different effective cross sections.

6. Instrument according to claim 5, **characterized in that** a ratio of the effective cross sections lies in a range of 4:1 to 9:1.

7. Instrument according to any one of the preceding claims, **characterized in that** the two fluid cylinders (98, 100) are pneumatic and/or hydraulic cylinders.

8. Instrument according to any one of the preceding claims, **characterized in that** the instrument (10) is designed for the removal of bone, cartilage or similar tissue (54), having a shank (18), which extends in a longitudinal direction (16) and carries on its distal end a cutting plate (20), which is disposed transversely of the longitudinal direction (16) or inclined relative to this longitudinal direction, **in that** the movably mounted tool is a cutting element (36) mounted displaceably on the shank (18) and carrying on its distal end a cutting edge (38), which is directed towards the cutting plate (20) and movable towards the cutting plate (20) for the cutting of tissue (54).

9. Instrument according to any one of the preceding claims, **characterized in that** in an inoperative position of the instrument (10), in which no actuating force is exerted by the force-transmitting and/or actuating mechanism (92, 128) in an actuating direction on the tool (36), a retaining force may be exerted on the tool (36) by the force-transmitting and/or actuating mechanism (92, 128) in a retaining direction opposite to the actuating direction.

10. Instrument according to claim 9, **characterized in that** the force-transmitting and/or actuating mechanism (92, 128) comprises an actuating member (94) for actuating the drive unit (92), **in that** the actuating member (94) is movable from an non-actuated basic position, in which the instrument (10) occupies the inoperative position, into a first activation position, in which the drive unit (92) occupies the first drive position, and into at least a second activation position, in which the drive unit occupies the second drive position.

11. Instrument according to claim 10, **characterized in that** the actuating member (94) is held under spring bias in the basic position.

12. Instrument according to any one of claims 10 or 11, **characterized in that** the actuating member (94) is mounted pivotably.

13. Instrument according to any one of claims 10 to 12, **characterized in that** in the first activation position a first resetting force acts upon the actuating member (94), **in that** in the second activation position a second resetting force acts, and **in that** the second resetting force is greater than the first resetting force.

14. Instrument according to claim 13, **characterized in that** the second resetting force is at least twice as great as the first resetting force.

15. Instrument according to any one of claims 13 or 14, **characterized in that** for generating the first and second resetting force there is provided a resetting unit (142, 146) comprising a first elastic resetting member (142) and a second elastic resetting member (146).

16. Instrument according to claim 15, **characterized in that** the first and/or the second resetting member is a spring element (142, 146).

17. Instrument according to any one of claims 15 or 16, **characterized in that** the resetting unit (142, 146) is configured to act directly or indirectly upon a control member (96) for controlling the drive unit (92) or upon the actuating member (94).

18. Instrument according to any one of claims 10 to 17, **characterized in that** a control member (96) is provided for controlling the drive unit (92) and that the actuating member (94) acts directly or indirectly upon the control member (96).

19. Instrument according to claim 18, **characterized in that** the control member (96) comprises at least one control valve.

20. Instrument according to claim 19, **characterized in that** the at least one control valve (96) has at least three different switching positions.

21. Instrument according to claim 19 or 20, **characterized in that** the control valve (96) comprises a valve plunger (138), **in that** on the valve body (137) there are provided at least one port connectable to a fluid source and two ports for each double-acting fluid cylinder (98, 100), that in a first position of the valve plunger (138) at least one of the fluid cylinders (98, 100) can be loaded with fluid in a retaining direction, **in that** in a second position of the valve plunger (138) at least one of the fluid cylinders (98, 100) can be loaded at both sides with differing fluid flows, and **in that** in a third position of the valve plunger (138) at least one of the fluid cylinders (98, 100) can be loaded with a fluid counter to the retaining direction and **in that** in the second position of the valve plunger (138) a fluid flow is distributed unevenly to the two ports of at least one fluid cylinder (98, 100).

22. Instrument according to claim 21, **characterized in that** the valve plunger (138) is provided with a plurality of annular grooves (190, 191, 192, 193, 194, 195) and in a valve body (137) in different switching positions defines different annular chambers, the ratios of cross section of which in dependence upon a fluid pressure are mutually adapted in such a manner that in the second switching position only a fraction of a maximum actuating force may be generated by the at least one fluid cylinder (98, 100).

23. Instrument according to any one of claims 19 to 22, **characterized in that** at least two control valves (96) are provided, each having at least two switching positions.

24. Instrument according to claim 23, **characterized in that** the at least two control valves (96) are manually separately actuable.

25. Instrument according to any one of the preceding claims, **characterized in that** on the tool part (14) at least one coding unit is provided for coding the nature and/or type of the tool part (14) and **in that** on the handling part (12) a decoding unit is provided for decoding the nature and/or type of the tool part (14).

26. Instrument according to claim 25, **characterized in that** the at least one coding unit comprises no or at least one projection protruding from the tool part (14) or at least one recess disposed in the tool part (14), that the decoding unit comprises an operating-mode switching member that corresponds to the at least one projection or the at least one recess, that the operating-mode switching member has at least a first and a second operating-mode position, and that the operating-mode switching member in accordance with the coding of the tool part (14) occupies one of the at least two operating-mode positions.

27. Instrument according to claim 26, **characterized in that** the at least two operating-mode positions are associated with maximum actuating forces of the drive unit (92).

28. Instrument according to claim 26 or 27, **characterized in that** the operating-mode switching member is coupled to the control member (96) and **in that** the operating-mode switching member in at least one operating-mode position directly or indirectly deactivates at least one switching position of the control member (96).

## Revendications

1. Instrument chirurgical (10) comprenant une partie de poignée de manoeuvre (12) et une partie d'outil (14), instrument
dans lequel la partie d'outil (14) comprend au moins un outil (36) monté de manière mobile, qui peut être actionné par l'intermédiaire d'un mécanisme de transmission de force et/ou d'actionnement (92, 128) pouvant être commandé à partir de la partie de poignée de manoeuvre (12), et le mécanisme de transmission de force et/ou d'actionnement (92, 128) comprend une unité d'entraînement (92) fonctionnant avec un fluide,
dans lequel à l'aide de l'unité d'entraînement (92), l'outil (36) monté de manière mobile peut, dans une première position d'entraînement, être sollicité par une première force d'actionnement, et dans au moins une deuxième position d'entraînement, être sollicité par au moins une deuxième force d'actionnement, et
dans lequel l'unité d'entraînement (92) comprend un entraînement linéaire, l'entraînement linéaire (92) comprenant deux vérins à fluide (98, 100) couplés, **caractérisé en ce que** les vérins à fluide peuvent être commandés de manière séparée.

2. Instrument selon la revendication 1, **caractérisé en ce qu'**à l'aide de l'unité d'entraînement (92), l'outil (36) monté de manière mobile peut, dans une troisième position d'actionnement, être sollicité par une troisième force d'actionnement.

3. Instrument selon la revendication 2, **caractérisé en ce que** la troisième force d'actionnement correspond à la somme de la première et de la deuxième force d'actionnement.

4. Instrument selon l'une des revendications précédentes, **caractérisé en ce qu'**un rapport de la première et de la deuxième force d'actionnement se situe dans une plage de 4:1 à 9:1.

5. Instrument selon l'une des revendications précédentes, **caractérisé en ce que** les deux vérins à fluide (98, 100) présentent des sections transversales actives différentes.

6. Instrument selon la revendication 5, **caractérisé en ce qu'**un rapport des sections transversales actives se situe dans une plage de 4:1 à 9:1.

7. Instrument selon l'une des revendications précédentes, **caractérisé en ce que** les deux vérins à fluide (98, 100) sont des vérins pneumatiques et/ou hydrauliques.

8. Instrument selon l'une des revendications précédentes, **caractérisé en ce que** l'instrument (10) est conçu pour retirer de l'os, du cartilage ou des tissus (54) similaires, avec un corps allongé en forme de tige (18), qui s'étend dans une direction longitudinale (16) et porte, à son extrémité distale, une lame ou plaquette de coupe (20) agencée transversalement à la direction longitudinale (16) ou inclinée par rapport à celle-ci, et **en ce que** l'outil monté mobile est un élément de coupe (36) monté coulissant sur le corps allongé en forme de tige (18), et qui, à son extrémité distale, porte un tranchant de coupe (38) dirigé vers la lame ou plaquette de coupe (20) et pouvant être ramené contre la lame ou plaquette de coupe (20) pour couper des tissus (54).

9. Instrument selon l'une des revendications précédentes, **caractérisé en ce que** dans une position de repos de l'instrument (10), dans laquelle le mécanisme de transmission de force et/ou d'actionnement (92, 128) n'exerce aucune force d'actionnement sur l'outil (36) dans une direction d'actionnement, il est possible d'exercer une force de maintien sur l'outil (36) à l'aide du mécanisme de transmission de force et/ou d'actionnement (92, 128), dans une direction de maintien opposée à la direction d'actionnement.

10. Instrument selon la revendication 9, **caractérisé en ce que** le mécanisme de transmission de force et/ou d'actionnement (92, 128) comprend un organe d'actionnement (94) pour actionner l'unité d'entraînement (92), et **en ce que** l'organe d'actionnement (94) peut, à partir d'une position initiale non actionnée dans laquelle l'instrument (10) prend la position de repos, être amené dans une première position d'action dans laquelle l'unité d'entraînement (92) prend la première position d'entraînement, et être amené dans au moins une deuxième position d'action dans laquelle l'unité d'entraînement prend la deuxième position d'entraînement.

11. Instrument selon la revendication 10, **caractérisé en ce que** l'organe d'actionnement (94) est maintenu dans la position initiale en y étant précontraint de façon élastique.

12. Instrument selon l'une des revendications 10 ou 11, **caractérisé en ce que** l'organe d'actionnement (94) est monté pivotant.

13. Instrument selon l'une des revendications 10 à 12, **caractérisé en ce que** dans la première position d'action, une première force de rappel agit sur l'organe d'actionnement (94), **en ce que** dans la deuxième position d'action, agit une deuxième force de rappel, et **en ce que** la deuxième force de rappel est plus grande que la première force de rappel.

14. Instrument selon la revendication 13, **caractérisé en ce que** la deuxième force de rappel est au moins égale au double de la première force de rappel.

15. Instrument selon l'une des revendications 13 ou 14, **caractérisé en ce que** pour produire la première et la deuxième force de rappel, il est prévu un unité de rappel (142, 146) comprenant un premier organe de rappel élastique (142) et un deuxième organe de rappel élastique (146).

16. Instrument selon la revendication 15, **caractérisé en ce que** le premier et/ou le deuxième organe de rappel est un élément de ressort (142, 146).

17. Instrument selon l'une des revendications 15 ou 16, **caractérisé en ce que** l'unité de rappel (142, 146) est conçu de manière à agir directement ou indirectement sur un organe de commande (96) destiné à commander l'unité d'entraînement (92), ou sur l'organe d'actionnement (94).

18. Instrument selon l'une des revendications 10 à 17, **caractérisé en ce qu'**il est prévu un organe de commande (96) pour commander l'unité d'entraînement (92), et **en ce que** l'organe d'actionnement (94) agit directement ou indirectement sur l'organe de commande (96).

19. Instrument selon la revendication 18, **caractérisé en ce que** l'organe de commande (96) comprend au moins une soupape de commande.

20. Instrument selon la revendication 19, **caractérisé en ce que** ladite au moins une soupape de commande (96) présente au moins trois positions de commutation différentes.

21. Instrument selon la revendication 19 ou la revendication 20, **caractérisé en ce que** la soupape de commande (96) comporte un poussoir de soupape (138), **en ce que** dans le corps de soupape (137) sont prévus au moins un raccord pouvant être relié à une source de fluide et deux raccords pour chaque vérin à fluide (98, 100) à double effet, **en ce que** dans une première position du poussoir de soupape (138), au moins l'un des vérins à fluide (98, 100) peut être alimenté en fluide dans une direction de maintien, **en ce que** dans une deuxième position du poussoir de soupape (138), au moins l'un des deux vérins à fluide (98, 100) peut être alimenté des deux côtés avec des écoulements de fluide différents, **en ce que** dans une troisième position du poussoir de soupape (138), au moins l'un des vérins à fluide (98, 100) peut être alimenté en fluide à l'encontre de la direction de maintien, et **en ce que** dans la deuxième position du poussoir de soupape (138), un écoulement de fluide est réparti de manière inégale sur les deux raccords d'au moins un vérin à fluide (98, 100).

22. Instrument selon la revendication 21, **caractérisé en ce que** le poussoir de soupape (138) est muni d'une pluralité de rainures annulaires (190, 191, 192, 193, 194, 195), et définit dans un corps de soupape (137), dans des positions de commutation différentes, des chambres annulaires différentes, dont les rapports de section transversale sont adaptés réciproquement les uns aux autres en fonction d'une pression de fluide, de façon telle que dans la deuxième position de commutation, seule une fraction d'une force d'actionnement maximale puisse être produite par ledit au moins un vérin à fluide (98, 100).

23. Instrument selon l'une des revendications 19 à 22, **caractérisé en ce qu'**il sont prévues au moins deux soupapes de commande (96) avec chacune au moins deux positions de commutation.

24. Instrument selon la revendication 23, **caractérisé en ce que** lesdites au moins deux soupapes de commande (96) peuvent être actionnées de manière manuelle séparée.

25. Instrument selon l'une des revendications précédentes, **caractérisé en ce que** sur la partie d'outil (14) il est prévu au moins une unité de codage pour coder la nature et/ou le type de la partie d'outil (14), et **en ce que** sur la partie de poignée de manoeuvre (12) il est prévu une unité de décodage pour décoder la nature et/ou le type de la partie d'outil (14).

26. Instrument selon la revendication 25, **caractérisé en ce que** ladite au moins une unité de codage ne présente pas ou présente au moins une protubérance en saillie de la partie d'outil (14) ou au moins un évidement agencé sur la partie d'outil (14), **en ce que** l'unité de décodage comprend au moins un organe de commutation de modes de fonctionnement correspondant à ladite au moins une protubérance ou ledit au moins un évidement, **en ce que** l'organe de commutation de modes de fonctionnement présente au moins une première et une deuxième position de modes de fonctionnement, et **en ce que** l'organe de commutation de modes de fonctionnement prend l'une desdites au moins deux positions de modes de fonctionnement conformément au codage de la partie d'outil (14).

27. Instrument selon la revendication 26, **caractérisé en ce que** lesdites au moins deux positions de modes de fonctionnement sont associées à des forces d'actionnement maximales de l'unité d'entraînement (92).

28. Instrument selon la revendication 26 ou la revendication 27, **caractérisé en ce que** l'organe de commutation de modes de fonctionnement est couplé à l'organe de commande (96), et **en ce que** l'organe de commutation de modes de fonctionnement, dans au moins une position de modes de fonctionnement, met directement ou indirectement hors de force au moins une position de commutation de l'organe de commande (96).
